# EUROPEAN PATENT SPECIFICATION

(11) **EP 4 514 413 B1**
(45) Date of publication and mention of the grant of the patent: **06.05.2026**
(21) Application number: 23720783.2
(22) Date of filing: 14.04.2023
(51) Int. Cl.: A61L 27/36

(54) **METHOD FOR THE PRODUCTION OF BIOLOGICAL TISSUE**
VERFAHREN ZUR HERSTELLUNG VON BIOLOGISCHEM GEWEBE
PROCÉDÉ DE PRODUCTION DE TISSU BIOLOGIQUE

(30) Priority: 29.04.2022 EP 22170910; 29.04.2022 EP 22170911; 04.05.2022 EP 22171615; 15.06.2022 EP 22179231; 15.06.2022 EP 22179232
(43) Date of publication of application: 05.03.2025
(73) Proprietor: BIOTRONIK AG, 8180 Bülach (CH)
(72) Inventor: RZANY, Alexander, 90449 Nuernberg (DE); HARDER, Claus, 91077 Dormitz (DE); LITSCHKO, Robert, 18069 Rostock (DE); MUELLER, Heinz, 18055 Rostock (DE)
(74) Representative: WSL Patentanwälte Partnerschaft mbB
(86) International application number: PCT/EP2023/059738
(87) International publication number: WO 2023/208607

(56) References cited:
- EP-A1- 3 156 083
- US-A1- 2010 011 564

## Description

The present invention is directed to a method for the preparation of a biodegradable tissue, the biogradable tissue obtained by said method, a medical implant comprising said biodegradable tissue and a device for preparation of the biodegradable tissue. A first aspect of the invention relates to a method for producing biodegradable and transparent biological tissue, preferably a collagen containing tissue. The (decellularized) biological tissue is first stabilized by a tissue water replacing material and then dried while applying an elevated pressure to the biological tissue. Also disclosed is a pressing unit/mold for stabilized drying of biological tissue (e.g. pericardium) under external pressure: The natively dried biological tissue is placed between two layers of a (technical) polyester fabric, which serves as drainage for the stabilizers and water. An open-cell polyurethane foam provides additional drainage while distributing the mechanical load. The two rigid outer forms are adjusted to a defined mechanical pressure (adjustable by the distance between the screwed outer forms) by means of screws. The remaining water is removed from the fabric by drying in the press mold in, for example, a climatic chamber.

A method for three-dimensional shaping by means of rigid molded bodies on both sides are known, for example, from US 8,136,218 B2. In the method described therein, the tissue is placed between two rigid shaped bodies and chemically crosslinked in this state. A method of manufacturing a prosthetic heart valve that includes processing dried biological material has been disclosed in US 8,105,375. According to the method disclosed therein, the biological tissue is fixed or crosslinked with an aldehyde-containing solution (e.g., glutaraldehyde or formaldehyde solution), and treated with at least one aqueous solution containing at least one biocompatible and non-volatile stabilizer prior to drying.

Chemical crosslinking by means of glutaraldehyde leads to inter- and intramolecular crosslinking in the collagen. Due to this the tissue cannot undergo enzymatic degradation. The cross-linked tissue is a non-biodegradable tissue. However, in some medical application a biodegradable tissue is required. However, the exemplary method of the prior art named above are subject to disadvantages. Thickness reduction is mostly based on chemical crosslinking under pressure, and thus on the principle of displacement of water from the original tissue, and an associated densification of the fibers of the tissue. However, if the tissue is limited from both sides by means of a fixed counterform as, for example, in the case of the use of two rigid molded bodies, there is no sufficient exchange surface for the water, and a very high pressure is necessary to be able to reduce this in turn.

The rigid molded bodies are also unable to compensate for the naturally always present inhomogeneity in the tissue thickness. In areas of higher tissue thickness, this also results in pressure peaks which cause partial fiber compaction and the associated stiffening of the tissue. In addition, the rigid mold bodies hinder the access of substances to the tissue.

Another problem of the prior art processes is that non-transparent tissue is obtained. However, in some application optical transparent tissue is required, e.g. for recognizing bleedings during an operation.

US 2010/011564 A1 discloses a mold assembly and a method for forming a prosthetic heart valve. EP 3 156 083 A1 discloses a method for thickness control and three-dimensional shaping of biological tissue during fixing.

With regard to the above-mentioned disadvantages of the prior art, an object of the first aspect of the first invention is to provide a method for the production of a biodegradable i.e. (substantially) non-crosslinked and optical transparent (collagenous) biological tissue.

The scope of protection is defined by the claims.The references to methods of treatment by therapy or surgery in the description are to be interpreted as references to implants of the present invention for use in those methods.

A method for the preparation of a biodegradable biological tissue is described in claim 1.

The biological (starting) tissue must be thoroughly cleaned and prepared prior to implantation. As far as possible, the tissue is modified in such a way that it is not recognized by the body as foreign tissue, has as little calcification as possible, and has as long a service life as possible.

According to the first aspect of the invention, the substantially non-crosslinked starting tissue, such as native biological tissue is processed to remove unwanted tissue residues.

Unless otherwise explicitly stated the (starting) tissue to be treated throughout the application is to be understood as biological tissue. Biological tissue preferably has an organizational level intermediate between cells and a complete organ. The biological (starting) tissue may be an autologous, xenogeneic or allogeneic tissue. In principle, all types of tissue e.g. from non-mammalian or mammalian tissue including human tissue can be used. The tissue may be derived from pig (porcine tissue), sheep, goat, horse (equine tissue), crocodile, kangaroo, ostrich, monkey, preferably primate, octopus, rabbit or cattle (bovine tissue). Tissue that can be used may be collagen containing tissue, pericardial tissue, skin, ligament, connective tissue, tendons, peritoneal tissue, dura mater, tela submucosa, in particular of the gastrointestinal tract, or pleura. Preferably the biological tissue is dura mater. The tissue can be in its native form or in a processed form or can comprise combinations thereof.

Autologous tissue (in medicine) refers to tissue that was isolated from the human or animal body and is to be re-transplanted elsewhere in the same human or animal body (i.e. originating from the same human or animal body or in other words donor and recipient are the same). The autologous tissue can be in its native form or in a processed form or can comprise combinations thereof. The autologous tissue to be used comprises chemically and/or biochemically crosslinkable groups.

Allogeneic tissue (in medicine) refers either to material that was isolated from a(nother) human or animal body that is genetically distinct from the human or animal body, but of the same species. Thus, allogeneic (also denoted as allogenic or allogenous) tissue is tissue that was isolated from a human or animal body which is different from the human or animal body where the implant is to be implanted. Allogeneic tissue can be not from the patient itself (but from a genetic different donor of the same species). Allogeneic here also includes hemiallogeneic (genetically different because of being derived from one parent of the same species and one parent from another species). The allogeneic tissue can be in its native form or in a processed form or can comprise combinations thereof. The allogenic tissue to be used comprises chemically and/or biochemically crosslinkable groups.

Xenogeneic tissue (in medicine) refers to tissue that was isolated from a human or animal body of a different (heterologous) species. Thus, xenogeneic (also known as xenogenous or xenogenic) tissue is material that was isolated form a human or animal body which is different from the human or animal body where the implant is to be implanted. Xenogeneic tissue may also refer to tissue based on human or animal donor cells (cells obtained from a or the human or animal donor) being cultivated in a bioreactor or being obtained via 3D printing. The xenogeneic material, e.g. tissue, can be in its native form, in a fixed form, in a processed form or can comprise combinations thereof.

The (starting) tissue is rinsed at least once, preferably several times, with a suitable solvent, in particular a buffered saline solution and/or an alcohol solution, before and optionally after a decellularization (if the tissue is decellularized). Buffered sodium chloride solutions and/or an ethanol solution are particularly advantageous.

One step of preparation is a preservation and stabilization of substantially non-crosslinked tissue, preferably native biological tissue, or pretreated, e.g. decellularized (native) tissue, which is characterized by an at least partial substitution of the (native) tissue water with a tissue water replacing material and a subsequent controlled drying. The process of the first aspect of the invention enables stabilization of (substantially) non-crosslinked tissue, preferably native biological tissue.

For the preservation and stabilization of essentially non-crosslinked, preferably native biological tissue, water plays a crucial role in the specific conformation of collagen molecules. A distinction is made between structural water, bound water and free water. Structural water, also called tightly bound water, is associated with stabilization of the triple helix structure. Intermolecular water molecules between triple helices and microfibrils are called bound water or contact water. Between the microfibrils and fibrils is free, unbound water. As water is removed, the collagen fibers lose their flexibility. If the process is limited to free, unbound water only, this process is reversible. However, if the drying process results in the removal of bound water molecules, the fiber network is irreversibly damaged. The absence of water causes a change in tissue conformation, which is accompanied by an increase in intra- and possibly also intermolecular hydrogen bonding. Complete rehydration of the fiber composite is thus precluded. The elastic properties of the collagen fibers are lost, and the tissue becomes brittle and fragile.

Consequently, the first aspect of the invention is based on a task to provide a preservation and stabilization process of essentially non-crosslinked, in particular native or decellularized biological tissue in order to enable i) any further processing and ii) at the same time also shaping directly in an substantially non-crosslinked or native state.

With the context of the first aspect of the invention, the term "substantially non-crosslinked" means that the corresponding tissue may be, for example, slightly pre- or partially crosslinked, but at least 50%, preferably at least 60%, more preferably at least 70%, still more preferably at least 80%, most preferably at least 90% of crosslinkable groups are still present in the tissue to be subjected to the processes according to the first aspect of the invention.

In order to enable structure-preserving drying of the tissue, i.e. preservation/stabilization, according to the first aspect of the invention a tissue water replacing material (e.g. glycerol and/or polyethylene glycol, if necessary as mixture(s)) is/are used, which stabilizes the collagen structure when water is removed. In this way, rehydration makes it possible to restore the tissue to its initial state.

In a stabilization step, the tissue is exposed to at least one solution containing glycerol and/or polyethylene glycol, wherein the tissue is exposed to either one of these solutions or to the two solutions sequentially in any order and composition as first and second solutions or to both solutions or even to multiple solutions with different molecular weights of PEG simultaneously as a mixture of solutions or sequentially in any order. When drying tissue, e.g., for storage or transportation of the tissue, the stabilization method is preferably carried out prior to drying.

The polyethylene glycol-containing solutions typically contain polyethylene glycol with an average molecular weight between 150 g/mol and 6000 g/mol, or a mixture thereof. As used herein, the term "between" also includes the upper and lower specified values. Thus, an average molecular weight between 150 g/mol and 6000 g/mol is intended to include 150 g/mol and 6000 g/mol.

**In** some embodiments, at least one polyethylene glycol-containing solution comprises polyethylene glycol having an average molecular weight between 150 g/mol and 200 g/mol, between 150 g/mol and 300 g/mol, between 200 g/mol and 300 g/mol, between 200 g/mol and 600 g/mol, between 200 g/mol and 400 g/mol, between 150 g/mol and 400 g/mol, or between 400 g/mol and 600 g/mol. According to a particularly preferred embodiment, the polyethylene glycol-containing solution provided alone or before or after a glycerol solution contains polyethylene glycol at or about 150 g/mol to 300 g/mol or at or about 200 g/mol (e.g., PEG200), and in an even more preferred embodiment, the polyethylene glycol-containing solution contains 40% PEG200 or about 40% PEG200.

The term "about" as used herein is intended to encompass a variation above and below the stated amount that would be expected in normal use, such as a variation of 5% or 10%. Glycerin may be added to any of the above stabilizing solutions to form a mixture, or it may be provided separately for stabilizing purposes, such as in aqueous solution.

In some embodiments, a subsequently applied polyethylene glycol-containing solution contains polyethylene glycol having a higher average molecular weight than a previously applied polyethylene glycol-containing solution. In some embodiments, the subsequently applied polyethylene glycol-containing solution contains polyethylene glycol having an average molecular weight between 200 g/mol and 6000 g/mol, or a mixture thereof. In some embodiments, the subsequently applied polyethylene glycol-containing solution comprises polyethylene glycol having an average molecular weight between 300 g/mol and 1500 g/mol, or a mixture thereof.

In some embodiments, the subsequently applied polyethylene glycol-containing solution comprises polyethylene glycol having an average molecular weight between 400 g/mol and 1200 g/mol, or a mixture thereof. In some embodiments, the subsequently applied polyethylene glycol-containing solution comprises polyethylene glycol having an average molecular weight between 400 g/mol and 800 g/mol, or a mixture thereof. In some embodiments, the subsequently applied polyethylene glycol-containing solution comprises polyethylene glycol having an average molecular weight between 400 g/mol and 600 g/mol, or a mixture thereof. In some embodiments, the subsequently applied polyethylene glycol-containing solution contains polyethylene glycol having an average molecular weight of 400 g/mol (PEG400) or about 400 g/mol.

Again, glycerol may be added to any of the above stabilizing solutions to form a mixture, or it may be provided separately as a stabilizing solution.

For example, too high temperatures (e.g., above about 85°C) during tissue preparation can cause denaturation and irreversible damage to the tissue. Too low temperatures can lead to a solution that is very viscous. Preferably, exposure to the stabilizing solutions may be at 37°C, temperatures from room temperature up to 60°C should are preferred.

The stabilization method may be performed after decellularization by immersing the tissue in a series of one or more stabilizing solutions of glycerol and/or polyethylene glycol to sufficiently saturate the tissue with stabilizing agents, and ultimately to produce a stable, dry tissue with a seamless joint/joint. Saturation times can vary, but typically take about 5 minutes to 2 hours or 5 minutes to 15 minutes, depending on the properties of the tissue.

The substantially non-crosslinked or native tissue, optionally decellularized tissue, is then preserved with a tissue water replacing material such as glycerol, polyethylene glycol (PEG) or a mixture of both, also comprising various mixtures of PEG, preferably with a group of substitutes consisting of glycerol, polyethylene glycol 200 and polyethylene glycol 400.

According to one embodiment of the first aspect of the invention, the biological tissue can be subjected to a decellularization step with a suitable decellularizing agent, e.g. surfactin and/or deoxycholic acid, DNase, etc., can be performed to generate a collagen-containing tissue matrix with reduced immune response in vivo. In a decellularization step, cell membranes, intracellular proteins, cell nuclei and other cellular components are almost completely removed from the tissue to obtain an extracellular matrix. In dura mater or pericardial tissue, the extracellular matrix is predominantly formed from collagen fibers. After decellularization, as many cellular components as possible are removed from the tissue and the biological material consists exclusively of extracellular matrix. In pericardial tissue, the extracellular matrix is predominantly formed from said collagen fibers. Cells and cellular components remaining in the tissue represent in particular a possible cause of undesired calcification of the biological implant material. Decellularization should be carried out so gently that the structure of the extracellular matrix and in particular the collagen fibers in the extracellular matrix remain as unaffected as possible, while on the other hand all cells and cellular components contained therein are removed from the tissue as completely as possible. The decellularization can be carried out in another way, for example by lysis of the cells or by osmotic digestion. The decellularization is preferably carried out by using a solution containing surfactin and/or deoxycholic acid.

After decellularization, as many cellular components as possible are removed from the tissue and the biological material consists exclusively of extracellular matrix. In pericardial tissue, the extracellular matrix is predominantly formed from said collagen fibers.

In one embodiment of the first aspect of the invention, alpha-gal epitopes may additionally be removed from the tissue in a further treatment step, which may be carried out after or before the optional decellularization step. Any suitable alpha-galactosidase can be used for such an additional treatment step, e.g., alpha-galactosidase from green coffee bean (GCB) or Cucumis melo.

The stabilized and optionally decellularized tissue is then pressed, e.g. between two rigid bodies using at least one, preferably two, permeable material layer(s) and at least one, preferably two pressure compensation layer(s), and dried. At the same time a 3D shape can be pressed into the biological tissue.

Essential for the drying is the drainage/removal of (tissue) water and the tissue water replacing material.

One, two or more permeable material layers, preferably made of a technical fabric, can be used. The at least one, preferably two, permeable material layer(s) may be an organic polymer layer (comprising polymer threads), preferably a polyester layer (comprising polyester threads). The at least one, preferably two, permeable material layer(s) has a mesh size or pore size enabling water and the tissue water replacing material to pass through the permeable material layer(s). This enables a sufficient drying the tissue with the crosslinking solution passing through the permeable material layer(s). The at least one, preferably two, permeable material layer(s) can have a mesh size or pore size of less than 60 µm, preferably ranging from 10 µm to 60 µm. Mesh sizes or pore sizes of less than 60 µm lead to even tissue surfaces. Mesh sizes or pore sizes larger than 60 µm lead to uneven tissue surfaces so that an imprinted structure on the surface of the biological tissue may be visible to the naked eye. Nevertheless, mesh sizes or pore sizes larger than 60 µm can be used to imprint structures on the surface of the biological tissue if this is desired, namely, whenever one wishes to imprint a technically functional surface on the tissue to be treated, such as, for example, a roughening of a surface or specific depressions in a surface, etc. The at least one, preferably two, permeable material layer can have a thickness of 40 µm to 70 µm. Preferably the tissue is sandwiched between two permeable material layer(s). The at least one, preferably two, permeable material layer(s) can have a 100-180 polymer, e.g. polyester, threads / cm.

The use of at least one permeable material layer makes it possible to remove the water contained in the tissue by applying comparatively little force and, at the same time, to reduce the tissue thickness considerably (from, for example, 200 µm initial tissue thickness to up to 50 µm or even 20 µm final thickness of the treated tissue, i.e. 10% of the initial thickness), while retaining the mechanical stability of the tissue itself.

In addition to the at least one, preferably two, permeable material layer(s) at least one, preferably two, pressure compensation layer(s) are be used. The pressure compensation layer can be permeable, but it does not have to be. A pressure compensation layer may be for example a compressible (polymer) material, e.g. a (polymer) foam, that is permeable to water and the hygroscopic exchange material, or an elastomer mat/silicone mat that is not permeable.

The pressure compensation layer, even if it is not permeable, at least supports the adequate distribution of the tissue water replacing material by its technical properties. The at least one, preferably two, pressure compensation layer(s) avoid(s) local stress peaks and enable a homogeneous thickness of the obtained tissue. Preferably the tissue is sandwiched between two permeable material layer(s) and the two permeable material layer(s) are sandwiched between two pressure compensation layers and the two pressure compensation layers are sandwiched between two (rigid) counterforms (via which an external pressure is applied). The at least one, preferably two, material layers and/or the at least one pressure compensation layer can be made of a hydrophilic material. The at least one pressure compensation layer can be a solid polymeric foam, preferably a polyurethane foam. The at least one pressure compensation layer can have a compression hardness of more than 60kPa preferably more than 66kPA, and/or a density of more than 40 kg/m³, preferably more than 46 kg/m³. The higher the compression hardness and bulk density are selected the higher is the pressure built up on the tissue. For example, an open-pored polyurethane foam as a permeable pressure compensation layer can be used, e.g. to match the natural inhomogeneity of the biological tissue (thickness homogeneity better than ±10µm) and to improve the mass transfer and avoid local mechanical stress peaks. The polyurethane foam can have a compression hardness of more than 60 kPa and/or a density of more than 40 kg/m³ and/or a thickness of more than 1cm, preferably 3 cm.

The use of one or more (permeable) pressure compensation layer(s), such as a compressible polymer foam, for force transmission guarantees the equalization of the thickness differences naturally occurring in the biological tissue - without causing local stress peaks that can lead to local stiffening of the tissue.

The use of the polyurethane foam as a pressure compensation layer and to transmit the force of the compressive load ensures compensation of the natural inhomogeneities of the pericardial tissue, avoiding local stress peaks. As a result, the resulting tissue has a particularly advantageous, and if required, extremely thin thickness homogeneity.

Another possibility of pressing uses a pressing structure, having from bottom to top the following parts:
- first (non-porous, porous or perforated) solid counterform, e.g. porous ceramic counterform,
- first pressure compensation layer, e.g. silicone layer or polyurethane foam,
- first permeable material layer, e.g. a polyester mesh
- the tissue to be treated,
- second permeable material layer, e.g. a polyester mesh,
- (optional) second pressure compensation layer, e.g. silicone layer or polyurethane foam,
- second (non-porous, porous or perforated) solid counterform, e.g. porous ceramic counterform.

For this purpose, a suitable device is provided for applying a constant pressure or a dynamic, periodic pulsatile pressure can be generated on the tissue, but over shorter and more frequent period cycles.

The drying step may be carried out in a climatic chamber. However, the stabilized tissue can be dried by placing the tissue, for example, in a suitable environment with constant low relative humidity or, for example, controllable humidity and/or temperature. For example, the stabilized tissue can be dried by placing the tissue in a climate chamber or desiccator and reducing the relative humidit, e.g. from 95% to 10% over 12 hours at 37°C.

This process according to the first aspect of the invention results in a biodegradable and transparent tissue, preferably pericardial tissue or dura mater. Furthermore, the obtained tissue is mechanically stable and flexible at the same time, therefore it can be sutured with itself or with another tissue or to a medical implant, like stent. The obtained tissue can be stored in a dried form. The obtained tissue can be rehydrated. The obtained biodegradable (non-crosslinked) tissue can be colonized or converted by endogenous cells (in contrast to non-biodegradable (crosslinked) tissue, which cannot be colonized or converted by endogenous cells.

A biodegradable (non-crosslinked) tissue could be used, for example, in cases where cellular ingrowth is preferred, such as in the treatment of a wound or burn with a porous matrix or when used as a means of sealing an implant or graft.

The obtained tissue can be punched out or cut, e.g. by laser cutting for example with a CO₂ laser.

The obtained tissue can be further sterilized, preferably using ethylene oxide.

The first aspect of the invention described herein using as an example a process for the preservation and stabilization of (substantially) non-crosslinked/ native biological tissue for use in implantation of the tissue in a human or animal body. For example, the first aspect of the invention is applicable to the preservation/stabilization of for example blood vessels, dura mater, connective tissue, cartilage, ligaments or skin. The obtained biodegradable and transparent biological (collagenous) tissue can be used for repairing tissue defects, preferably dura mater defects. The obtained biodegradable and transparent biological (collagenous) tissue can be used as tissue patch or covering materials for a medical implant like a covered stent.

With regard to an implant, the aforementioned problem is further solved by an implant containing biological tissue which has been subjected to the process steps described above.

The medical implant may be a (cardio)vascular implant, a endovascular prostheses, an endoprostheses, an esophageal implant, a bile duct implant, a dental implant, an orthopedic implant, a sensory implant or a neurological implant a microchip containing implant, a stent, a vascular stent, a drug eluting stent, a pulmonary valve stent, a bile duct stent, a peripheral stent, a mitral stent, a stent graft, a drug eluting stent, a covered stent, a vascular patch, a tissue patch, a venous valve, a tooth implant, a bone implant, a glucose sensor implant, a neurostimulator, a cochlear implant, an endoprostheses for closing persistent foramen ovale, an endoprostheses for closing an atrial septal defect, a left atrial appendage closure device, a pacemaker, a leadless pacemaker, a defibrillator, a prosthetic heart valve, preferably a TAVI/TAVR valve.

Stents have a body in the form of a possibly perforated tubular or hollow cylindrical support structure, which is open at both longitudinal ends. The support structure of the stent may be composed of individual meshes formed by zigzag or meander-shaped webs. The tubular support structure of such an endoprosthesis is inserted into the vessel to be treated and serves to support the vessel. A covered stent usually consists of collagenous (i.e. collagen containing) tissue components integrated into a self-expanding or mechanically expandable stent (e.g., balloon-expandable) or support structure. A stent may be covered on the outer an/or on the inner side with the treated biological tissue according to the first aspect of the invention. Stents are particularly frequently used as implants for the treatment of stenoses (narrowing of blood vessels).

Stent graft(s)" are stents that contain a covering, such as biological tissue, on or in their (grid-like) basic structure. A stent graft is used, for example, to support weak points in arteries, esophagus or bile ducts, for example in the area of an aneurysm or a rupture of the vessel wall (so-called bail-out device), in particular as an emergency stent.

The following implementation of the first aspect of the invention by way of examples and embodiments is explained below:

### Example A

An exemplary process shown Example A describes the production of a biodegradable, transparent and dry biological collagen-containing tissue based on porcine pericardium for e.g. replacement of the dura mater.

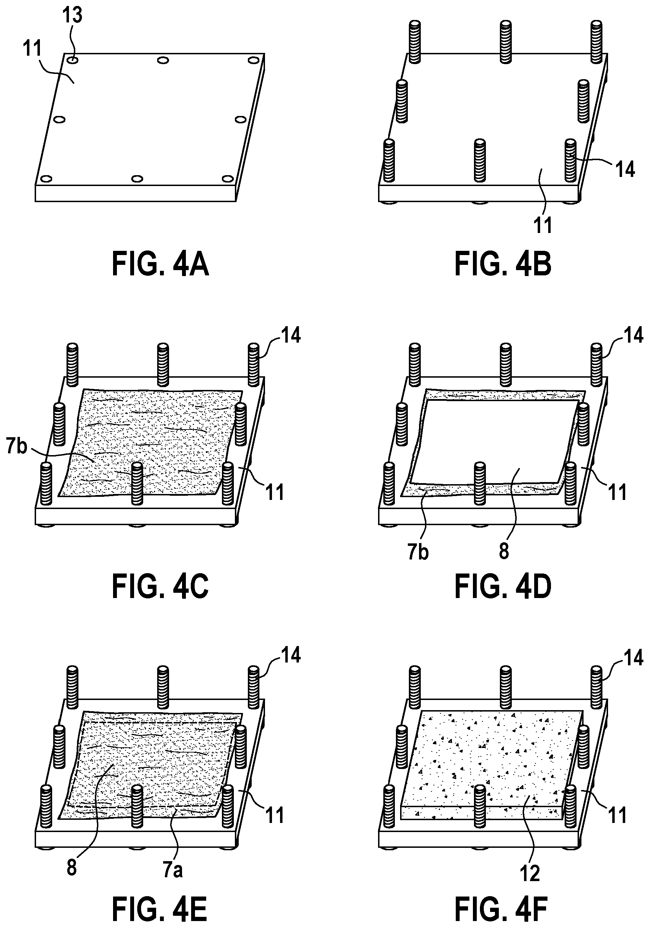

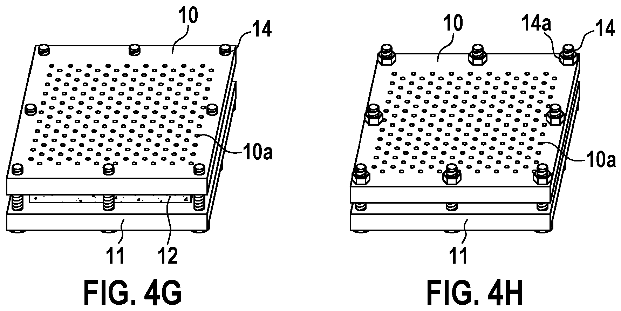

Method for preparing a biodegradable biological tissue comprising the following method steps
1) providing a pericardial tissue, e.g. from a pig, which is freshly collected at the slaughterhouse and stored for 2 h at 4°C in a suitable storage solution, e.g. EDTA/isopropanol or saline;
2) rinsing the pericardial tissue with saline (e.g. 0.9%) preferably three times for 5 min;
3) removing fat and/or connective tissue from the pericardial tissue in saline (0.9%) and is followed by gross cutting to approximately 12 cm x 8 cm;
4) rinsing the pericardial tissue with saline (e.g. 0.9%) preferably with gentle mechanical agitation;
5) stabilizing the pericardial tissue (e.g. 12x12cm2) in a glycerol solution (e.g. 100 ml of 30% glycerol in ultrapure water for 15 minutes, preferably with gentle agitation)
6) stabilizing the pericardial tissue in a polyethylene glycol solution (e.g. 100 ml of 40% PEG 200 in ultrapure water for 15 minutes, preferably with gentle agitation),
7) stabilizing pericardial tissue in a polyethylene glycol solution (e.g. 100 ml of 40% PEG 400 in ultrapure water for 15 minutes, preferably with gentle agitation),
8) (wrinkle-free) positioning of a first permeable material layer (7b), e.g. a first polyester mesh, on a first rigid counterform (11) (see Fig. 4C);
9) (wrinkle-free) positioning of the stabilized pericardial tissue (8) on the first permeable material layer (7b), optionally followed by a smoothing out of any air bubbles present in the pericardial tissue (8) (see Fig. 4D);
10) (wrinkle-free) positioning of a second permeable material layer (7a), e.g. a second polyester mesh, on the pericardial tissue (8) (see Fig. 4E);
11) positioning of a pressure compensation layer (12), e.g. an open porous polyurethane foam, on the second permeable material layer (7a) (see Fig. 4F);
12) providing of a second (upper) rigid counterform (10) which is porous or has perforations (10a) (see Fig. 4G);
13) fixation of the two rigid counterforms (10, 11) by means of screws; adjustment of the desired pressure via the counterform spacing (see Fig. 4H);
14) mounting the stabilized biological tissue in a press mold and placing it in a climatic chamber for drying,
15) pre-drying the stabilized biological tissue in the climate chamber by reducing the relative humidity from 95% to 10% over 24h at 40°C
16) drying of the pre-dried stabilized biological tissue in a climatic chamber by maintaining the relative humidity at 10% for another 24 h at 40°C.
17) removing the dried tissue from the press mold
18) optionally cutting of the dried planar tissue pieces into a desired shape by e.g. laser cutting with CO₂ laser
19) sterilizing the dried tissue e.g. by using ethylene oxide

Instead of step 4) the following steps 4a to 4g) can be performed:
4a) rinsing the pericardial tissue with NaCl (0.9%) preferably with gentle agitation,
4b) Decellularization of the pericardial tissue in surfactin-deoxycholic acid solution (e.g. 100 ml 0.06% surfactin and 0.5% deoxycholic acid in 0.9% NaCl for 22 h at 37°C by changing of the solution after every 2 h)
4c) rinsing the pericardial tissue with NaCl (0.9%) preferably 6x for 10 minutes preferably with gentle agitation
4d) Decellularization the pericardial tissue in DNase solution or DNase and GCB solution: e.g. for 2d at 37°C
4e) rinsing the decellularized pericardial tissue with NaCl (0.9%) with TRIS (pH 11) e.g. 4x for 10 minutes under gentle agitation
4f) rinsing the decellularized pericardial tissue with ethanol solution e.g in 100 ml 70% ethanol solution at 37°C for 15 minutes
4g) rinsing the decellularized pericardial tissue with NaCl (0.9%) e.g. 6x for 10 minutes under gentle agitation

The biological tissue is first mechanically prepared (process steps 1-4). The pericardium can also come from cattle, horses or other animals. Other tissue matrices containing collagen from, for example, the intestine or skin can also be used in an analogous manner.

Similarly, previously decellularized biological tissues can also be used, which reduces the body's immune response to the implant, resulting in better healing in dura mater replacement. For example, when such tissue is used, the chain of process steps 4a-g replaces process step 4 (see process diagram below).

The biological tissue is subsequently stabilized with the hygroscopic substitutes glycerol, polyethylene glycol 200 and polyethylene glycol 400 (process steps 5-7). The stabilizers already replace part of the water contained in the tissue and attach themselves to the fiber proteins in such a way that their structure is retained when the water is later removed by drying, i.e. they do not denature. As a result, the tissue remains optimally biodegradable and also mechanically flexible for the application, i.e. in the case of dura mater replacement, good adaptation to the wound is possible. The stabilizers can optionally be used in various combinations or selectively.

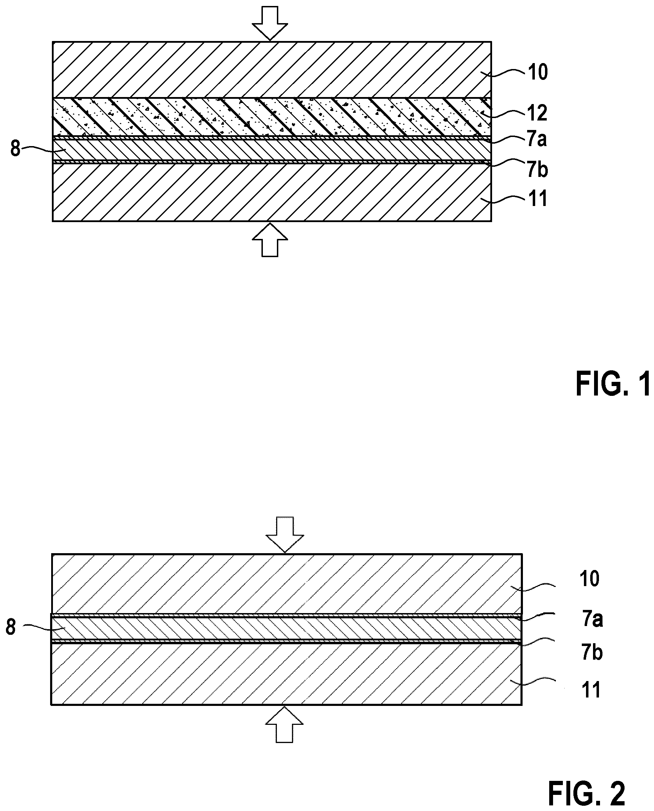

Crucial to the solution according to the first aspect of the invention is the subsequent drying in a compression mold under constant external mechanical pressure using drainage (process steps 8-17 and Figures 1 and 2).

The adaptation of the fabric properties such as flexibility, stability and transparency is mainly achieved by drying under pressure in combination with the use of a permeable technical fabric layer of, for example, polyester fabric acting as a drainage. Via this drainage, excess stabilizers contained in the stabilized fabric as well as residual stored water can be removed by applying comparatively little force and the fabric can thus be strongly compacted (in the case of porcine pericardium from 200µm to up to 50µm thickness). Polyester sieve fabrics with 100 to 180 threads/cm are particularly suitable. The mesh size ranges from 10µm to 60µm, with a thickness of the technical fabric of approx. 40µm to 70µm being suitable. Larger mesh sizes are also suitable with regard to fabric exchanges, but the imprinted structure on the surface of the biological fabric is then visible to the naked eye. The stabilized fabric is always sandwiched between two layers of the technical fabric to allow stock removal on both sides.

An additional open-pored polyurethane foam as a permeable pressure equalization layer can optionally be used to match the natural inhomogeneity of the biological base fabric (thickness homogeneity better than ±10 µm), improve stock removal and avoid local mechanical stress peaks. Usable polyurethane foams have a compression hardness >60 kPa and a density >40 kg/m³ at a thickness of 3cm.

The compressive load is applied, for example, within rigid outer molds. These are either porous (e.g. ceramics) or provided with holes or bores to allow the removal of water and excess stabilizers. Compression of the rigid outer molds is achieved by screws or clamps attached to the edge. The resulting mechanical pressure can be controlled by the plate spacing.

By continuously reducing the plate spacing e.g., by means of suitable screws and nuts, and the associated compression of the foam, the final thickness of the pericardial tissue can be specifically adjusted to the requirements of a subsequent application, in particular for a medical application. During compression, a pressure load is applied to the stabilized biological tissue. Excess stabilizers contained in the pericardial tissue, along with residual water, are removed via drainage and the fibrous proteins become more compacted with increasing pressure. This removes free areas between the fibrous proteins, which changes the optical properties and makes the tissue, which is opaque in its initial state, transparent. Possible compression pressures are in the range above 0.01 kg/cm2, with practically no upper limit.

The decisive factor for the solution according to the first aspect of the invention is the complete drying of the fabric within the compression mold under constant external pressure. Drying is carried out, for example, in two stages under controlled conditions in a climatic chamber: first, reduction of the relative humidity from 95% to 10% over 24 hours at 40°C and then another minimum of 24 hours at 10% relative humidity and 40°C for complete drying. Drying can also be done over longer periods of time or by other methods (air drying, oven drying).

According to the first aspect of the invention a stabilized dried tissue still comprising chemically and/or biochemically crosslinkable groups, preferably having a proportion of crosslinkable groups in the tissue to be treated compared to non-crosslinkable groups of more than 50%, can be obtained. This stabilized dried tissue is still biodegradable and can be colonized by cells. In contrast crosslinked tissue, e.g. crosslinked by the use of glutaraldehyde, (comprising much less than 50% chemically and/or biochemically crosslinkable groups) is not biodegradable and cannot be colonized by cells.

Biodegradable means that over a predetermined period of time the tissue can be absorbed by the human or animal body where it is to be implanted or it is converted into a degradation product by the human or animal body. The predetermined period of time may be less than 1 year preferably less than 1 month (but more than 2 days). For example, biodegradable can mean that tissue can be enzymatically degraded at the implantation site, e.g. by proteases. Preferably, the biodegradable tissue is to be biodegraded (bioabsorbed) once the traumatised tissue at the implantation site, e.g. a vessel, has healed the implanted tissue is no longer needed.

Native biological tissue is incubated in stabilizers in order to maintain biodegradability and mechanical flexibility. The biological tissue pre-treated in this way is then mechanically pressed in a compression mold in technical fabrics and open-cell foams for drainage. This densifies the fiber structure of the fabric, forms physical bonds between the protein fibers, removes excess stabilizers and, in particular, eliminates voids between the protein fibers, resulting in the required optical transparency of the material.

Chemical crosslinking by means of glutaraldehyde leads to inter- and intramolecular crosslinking in the collagen. Due to this the tissue cannot undergo enzymatic degradation. The cross-linked tissue is a non-biodegradable tissue.

However, the prior art named above are subject to disadvantages. Thickness reduction is mostly based on chemical crosslinking under pressure, and thus on the principle of displacement of water from the original tissue, and an associated densification of the fibers of the tissue. However, if the tissue is limited from both sides by means of a fixed counterform as, for example, in the case of the use of two rigid molded bodies, there is no sufficient exchange surface for the water, and a very high pressure is necessary to be able to reduce this in turn. The rigid molded bodies are also unable to compensate for the naturally always present inhomogeneity in the tissue thickness. In areas of higher tissue thickness, this also results in pressure peaks which cause partial fiber compaction and the associated stiffening of the tissue. In addition, the rigid mold bodies hinder the access of substances to the tissue.

Consequently, the invention is based on a task to provide a preservation and stabilization process of essentially non-crosslinked, in particular native or decellularized biological tissue in order to enable i) any further processing and ii) at the same time also shaping directly in an substantially non-crosslinked or native state.

With the context of the second aspect of the invention, the term "substantially non-crosslinked" means that the corresponding tissue may be, for example, slightly pre- or partially crosslinked, but at least 50%, preferably at least 60%, more preferably at least 70%, still more preferably at least 80%, most preferably at least 90% of crosslinkable groups are still present in the tissue to be subjected to the processes according to the second aspect of the invention.

In order to enable structure-preserving drying of the tissue, i.e. preservation/stabilization, according to the second aspect of the invention a tissue water replacing material (e.g. glycerol and/or polyethylene glycol, if I as mixture(s) is/are used, which stabilizes the collagen structure when water is removed. In this way, rehydration makes it possible to restore the tissue to its initial state.

In a stabilization step, the tissue is exposed to at least one solution containing glycerol and/or polyethylene glycol, wherein the tissue is exposed to either one of these solutions or to the two solutions sequentially in any order and composition as first and second solutions or to both solutions or even to multiple solutions with different molecular weights of PEG simultaneously as a mixture of solutions or sequentially in any order. When drying tissue, e.g., for storage or transportation of the tissue, the stabilization method is preferably carried out prior to drying.

The polyethylene glycol-containing solutions typically contain polyethylene glycol with an average molecular weight between 150 g/mol and 6000 g/mol, or a mixture thereof. As used herein, the term "between" also includes the upper and lower specified values. Thus, an average molecular weight between 150 g/mol and 6000 g/mol is intended to include 150 g/mol and 6000 g/mol.

In some embodiments, at least one polyethylene glycol-containing solution comprises polyethylene glycol having an average molecular weight between 150 g/mol and 200 g/mol, between 150 g/mol and 300 g/mol, between 200 g/mol and 300 g/mol, between 200 g/mol and 600 g/mol, between 200 g/mol and 400 g/mol, between 150 g/mol and 400 g/mol, or between 400 g/mol and 600 g/mol. According to a particularly preferred embodiment, the polyethylene glycol-containing solution provided alone or before or after a glycerol solution contains polyethylene glycol at or about 150 g/mol to 300 g/mol or at or about 200 g/mol (e.g., PEG200), and in an even more preferred embodiment, the polyethylene glycol-containing solution contains 40% PEG200 or about 40% PEG200.

The term "about" as used herein is intended to encompass a variation above and below the stated amount that would be expected in normal use, such as a variation of 5% or 10%. Glycerin may be added to any of the above stabilizing solutions to form a mixture, or it may be provided separately for stabilizing purposes, such as in aqueous solution.

In some embodiments, a subsequently applied polyethylene glycol-containing solution contains polyethylene glycol having a higher average molecular weight than a previously applied polyethylene glycol-containing solution. In some embodiments, the subsequently applied polyethylene glycol-containing solution contains polyethylene glycol having an average molecular weight between 200 g/mol and 6000 g/mol, or a mixture thereof. In some embodiments, the subsequently applied polyethylene glycol-containing solution comprises polyethylene glycol having an average molecular weight between 300 g/mol and 1500 g/mol, or a mixture thereof.

In some embodiments, the subsequently applied polyethylene glycol-containing solution comprises polyethylene glycol having an average molecular weight between 400 g/mol and 1200 g/mol, or a mixture thereof. In some embodiments, the subsequently applied polyethylene glycol-containing solution comprises polyethylene glycol having an average molecular weight between 400 g/mol and 800 g/mol, or a mixture thereof. In some embodiments, the subsequently applied polyethylene glycol-containing solution comprises polyethylene glycol having an average molecular weight between 400 g/mol and 600 g/mol, or a mixture thereof. In some embodiments, the subsequently applied polyethylene glycol-containing solution contains polyethylene glycol having an average molecular weight of 400 g/mol (PEG400) or about 400 g/mol.

Again, glycerol may be added to any of the above stabilizing solutions to form a mixture, or it may be provided separately as a stabilizing solution.

For example, too high temperatures (e.g., above about 85°C) during tissue preparation can cause denaturation and irreversible damage to the tissue. Too low temperatures can lead to a solution that is very viscous. Preferably, exposure to the stabilizing solutions may be at 37°C, temperatures from room temperature up to 60°C should are preferred.

The stabilization method may be performed after decellularization by immersing the tissue in a series of one or more stabilizing solutions of glycerol and/or polyethylene glycol to sufficiently saturate the tissue with stabilizing agents, and ultimately to produce a stable, dry tissue with a seamless joint/joint. Saturation times can vary, but typically take about 5 minutes to 2 hours or 5 minutes to 15 minutes, depending on the properties of the tissue.

The substantially non-crosslinked or native tissue, optionally decellularized tissue, is then preserved with a tissue water replacing material such as glycerol, polyethylene glycol (PEG) or a mixture of both, also comprising various mixtures of PEG, preferably with a group of substitutes consisting of glycerol, polyethylene glycol 200 and polyethylene glycol 400.

The stabilized and optionally decellularized tissue is then pressed, e.g. between two rigid bodies using at least one, preferably two, permeable material layer(s) and at least one, preferably two pressure compensation layer(s), and dried. At the same time a 3D shape can be pressed into the biological tissue.

Essential for the drying is the drainage/removal of (tissue) water and the tissue water replacing material.

One, two or more permeable material layers, preferably made of a technical fabric, are used. The at least one, preferably two, permeable material layer(s) may be an organic polymer layer (comprising polymer threads), preferably a polyester layer (comprising polyester threads). The at least one, preferably two, permeable material layer(s) has a mesh size or pore size enabling water and the tissue water replacing material to pass through the permeable material layer(s). This enables a sufficient drying the tissue with the crosslinking solution passing through the permeable material layer(s). The at least one, preferably two, permeable material layer(s) can have a mesh size or pore size of less than 60 µm, preferably ranging from 10 µm to 60 µm. Mesh sizes or pore sizes of less than 60 µm lead to even tissue surfaces. Mesh sizes or pore sizes larger than 60 µm lead to uneven tissue surfaces so that an imprinted structure on the surface of the biological tissue may be visible to the naked eye. Nevertheless, mesh sizes or pore sizes larger than 60 µm can be used to imprint structures on the surface of the biological tissue if this is desired, namely, whenever one wishes to imprint a technically functional surface on the tissue to be treated, such as, for example, a roughening of a surface or specific depressions in a surface, etc. The at least one, preferably two, permeable material layer can have a thickness of 40 µm to 70 µm. Preferably the tissue is sandwiched between two permeable material layer(s). The at least one, preferably two, permeable material layer(s) can have a 100-180 polymer, e.g. polyester, threads / cm.

The use of at least one permeable material layer it possible to remove the water contained in the tissue by applying comparatively little force and, at the same time, to reduce the tissue thickness considerably (from, for example, 200 µm initial tissue thickness to up to 50 µm or even 20 µm final thickness of the treated tissue, i.e. 10% of the initial thickness), while retaining the mechanical stability of the tissue itself.

In addition to the at least one, preferably two, permeable material layer(s) at least one, preferably two, pressure compensation layer(s) are used. The pressure compensation layer can be permeable, but it does not have to be. A pressure compensation layer may be for example a compressible (polymer) material, e.g. a (polymer) foam, that is permeable to water and the hygroscopic exchange material, or an elastomer mat/silicone mat that is not permeable.

The pressure compensation layer, even if it is not permeable, at least supports the adequate distribution of the tissue water replacing material by its technical properties. The at least one, preferably two, pressure compensation layer(s) avoid(s) local stress peaks and enable a homogeneous thickness of the obtained tissue. Preferably the tissue is sandwiched between two permeable material layer(s) and the two permeable material layer(s) are sandwiched between two pressure compensation layers and the two pressure compensation layers are sandwiched between two (rigid) counterforms (via which an external pressure is applied). The at least one, preferably two, material layers and/or the at least one pressure compensation layer can be made of a hydrophilic material. The at least one pressure compensation layer can be a solid polymeric foam, preferably a polyurethane foam. The at least one pressure compensation layer can have a compression hardness of more than 60kPa preferably more than 66kPA, and/or a density of more than 40 kg/m³, preferably more than 46 kg/m³. The higher the compression hardness and bulk density are selected the higher is the pressure built up on the tissue. For example, an open-pored polyurethane foam as a permeable pressure compensation layer can be used, e.g. to match the natural inhomogeneity of the biological tissue (thickness homogeneity better than ±10µm) and to improve the mass transfer and avoid local mechanical stress peaks. The polyurethane foam can have a compression hardness of more than 60 kPa and/or a density of more than 40 kg/m3 and/or a thickness of more than 1cm, preferably 3 cm.

The use of one or more (permeable) pressure compensation layer(s), such as a compressible polymer foam, for force transmission guarantees the equalization of the thickness differences naturally occurring in the biological tissue - without causing local stress peaks that can lead to local stiffening of the tissue.

The use of the polyurethane foam as a pressure compensation layer and to transmit the force of the compressive load ensures compensation of the natural inhomogeneities of the pericardial tissue, avoiding local stress peaks. As a result, the resulting tissue has a particularly advantageous, and if required, extremely thin thickness homogeneity.

Another possibility of pressing uses a pressing structure, having from bottom to top the following parts:
- first (non-porous, porous or perforated) solid counterform, e.g. porous ceramic counterform,
- (optional) first pressure compensation layer, e.g. silicone layer or polyurethane foam,
- first permeable material layer, e.g. a polyester mesh
- the tissue to be treated,
- second permeable material layer, e.g. a polyester mesh,
- (optional) second pressure compensation layer, e.g. silicone layer or polyurethane foam,
- second (non-porous, porous or perforated) solid counterform, e.g. porous ceramic counterform.

For this purpose, a suitable device is provided for applying a constant pressure or a dynamic, periodic pulsatile pressure can be generated on the tissue, but over shorter and more frequent period cycles.

The drying step may be carried out in a climatic chamber. However, the stabilized tissue can be dried by placing the tissue, for example, in a suitable environment with constant low relative humidity or, for example, controllable humidity and/or temperature. For example, the stabilized tissue can be dried by placing the tissue in a climate chamber or desiccator and reducing the relative humidity, e.g. from 95% to 10% over 12 hours at 37°C.

This process according to the second aspect of the invention results in a biodegradable and transparent tissue, preferably pericardial tissue or dura mater. Furthermore, the obtained tissue is mechanically stable and flexible at the same time, therefore it can be sutured with itself or with another tissue or to a medical implant, like stent. The obtained tissue can be stored in a dried form. The obtained tissue can be rehydrated. The obtained biodegradable (non-crosslinked) tissue can be colonized or converted by endogenous cells (in contrast to non-biodegradable (crosslinked) tissue, which cannot be colonized or converted by endogenous cells).

A biodegradable (non-crosslinked) tissue could be used, for example, in cases where cellular ingrowth is preferred, such as in the treatment of a wound or burn with a porous matrix or when used as a means of sealing an implant or graft.

The obtained tissue can be punched out or cut, e.g. by laser cutting for example with a CO₂ laser. The obtained tissue can be further sterilized, preferably using ethylene oxide.

The second aspect of the invention described herein using as an example a process for the preservation and stabilization of (substantially) non-crosslinked/ native biological tissue for use in implantation of the tissue in a human or animal body. For example, the second aspect of the invention is applicable to the preservation/stabilization of for example blood vessels, dura mater, connective tissue, cartilage, ligaments or skin. The obtained biodegradable and transparent biological (collagenous) tissue can be used for repairing tissue defects, preferably dura mater defects. The obtained biodegradable and transparent biological (collagenous) tissue can be used as tissue patch or covering materials for a medical implant like a covered stent.

With regard to an implant, the aforementioned problem is further solved by an implant containing biological tissue which has been subjected to the process steps described above.

The medical implant may be a (cardio)vascular implant, an endovascular prosthesis, an endoprostheses, an esophageal implant, a bile duct implant, a stent, a vascular stent, a drug eluting stent, a pulmonary valve stent, a bile duct stent, a peripheral stent, a mitral stent, a stent graft, a drug eluting stent, a covered stent.

Stents have a body in the form of a possibly perforated tubular or hollow cylindrical support structure, which is open at both longitudinal ends. The support structure of the stent may be composed of individual meshes formed by zigzag or meander-shaped webs. The tubular support structure of such an endoprosthesis is inserted into the vessel to be treated and serves to support the vessel. A covered stent usually consists of collagenous (i.e. collagen containing) tissue components integrated into a self-expanding or mechanically expandable stent (e.g., balloon-expandable) or support structure. A stent may be covered on the outer an/or on the inner side with the treated biological tissue according to the second aspect of the invention. Stents are particularly frequently used as implants for the treatment of stenoses (narrowing of blood vessels).

Stent graft(s) are stents that contain a covering, such as biological tissue, on or in their (grid-like) basic structure. A stent graft is used, for example, to support weak points in arteries, esophagus or bile ducts, for example in the area of an aneurysm or a rupture of the vessel wall (so-called bail-out device), in particular as an emergency stent.

Furthermore, it should be noted in general that collagen-containing biological materials such as pericardium can exhibit a fundamentally very similar mechanical behavior due to the microstructural composition consisting of an irregular network of collagen fibrils. When the biological material is stretched in the planar plane, the mechanical stress can initially increase very slowly with the stretch, since the collagen fibers can initially be aligned along the direction of the force. After alignment of the fibers, the collagen fibers can support mechanical load and a linear-elastic increase in mechanical stress with strain can occur. Above a certain mechanical stress, failure of progressively more collagen fibrils occurs until complete mechanical failure of the material occurs.

An associated elongation at break and stress at break may depend on a variety of possible initial parameters (such as tissue type, thickness, homogeneity and internal fiber distribution in the native starting material, etc.) and on a variety of process parameters (such as mechanical stress during preparation, crosslinking under mechanical tensile load, etc.). Collagen-containing tissues processed in this way typically exhibit elongations at break of 5-15%, with a maximum of about 30%.

For the covering of a stent 1, it must be ensured that no mechanical failure of the biological tissue covering due to the formation of substantial holes occurs during implantation and application of the stent. Given elongations at rupture of the biological material up to a maximum of about 30%, this may not be guaranteed or possible for typical balloon-expandable coronary stents. For example, the cover of a covered stent in current clinical practice with a cover made of a polymer can be stretched by about 250% when dilated from 6 French in the crimped state to 5 mm in the dilated state. Consequently, the required elongation at break may be about an order of magnitude greater than the elongation at break of typical collagen-containing biological materials, such as pericardium.

Alternatively, the tubing material according to a further embodiment may be coated on the inner or outer side with a medicament. The pericardium may originate from pigs, from other animals or from the patient to be treated himself (autologous pericardium).

The pericardial tissue tube may contain shape-stabilizing elements, such as a self-expandable NiTi stent or mesh. The pericardial tubing may alternatively include temporary shape stabilizing elements, such as e.g. a stent, scaffold or mesh consisting of a biodegradable polymer or a biodegradable metal, preferably magnesium or a magnesium alloy.

The bypass material may consist of a thin-walled self-expandable NiTi stent (or mesh), which has a pericardial layer on the inside and outside, so that the stent itself is has no contact with the biological environment. In a broader sense, this is a "pericardium covered stent."

The bypass material may consist of autologous pericardium previously removed from the patient's body. The advantage of this procedure is the reduction of the expected rejection reaction.

The bypass material may consist of cross-linked pericardium, which is formed into a tube using a sutureless joining technique.

In a specific embodiment, these pericardial tissue tubes may be connected to a (biodegradable) support structure, e.g. a biodegradable magnesium alloy scaffold to form a temporarily stabilized magnesium scaffold. The magnesium scaffold can be used for degradation control or to protect the tube against process-related influences.

The support structure may be fixed between two tissue tubes. An inner and outer tube may be connected. The solutions according to the second aspect of the invention described herein use as material a biological tissue preferably pericardium, or in general a (predominantly) collagen-containing biological matrix (e.g. intestinal tissue, tunica mucosa, ...). The starting material can be present in a (coherent) planar (two-dimensional) structure, Likewise, the biological species (porcine. Bovine, ovine, autologous pericardium, ...) is of no importance for the solutions according to the second aspect of the invention. In principle, it is possible, with a high process effort, to start from native biological tissue, i.e. without chemical fixation with e.g. glutaraldehyde, which are stored in a moist state. A very high effort is necessary to produce molded parts from the moist native fabric, since internal mechanical stress during cutting leads to unpredictable geometric distortions during cutting.

For the concrete technical implementation of the solutions according to the second aspect of the invention, it is possible to start from a native dried pericardium (or, in general, a native dried collagen-containing biological matrix). Only in this state is it possible to cut the pericardium geometrically stable and in small bypass dimensions necessary for required processing.

By forming an overlap of the native dried tissue (parts), it is possible to obtain a tissue-to-tissue bond, which allows a mechanical connection without the need for additional seams (also named seamless connection sutureless joining). The direct chemical-mechanical tissue connection saves considerable space, as additional connections by seams or individual nodes become superfluous. In addition, implementation in a production process is much easier or even economically feasible.

The following implementation of the second aspect of the invention by way of examples and embodiments is explained below:

### Example C (not according to the claimed invention)

The following exemplary process describes step-by-step the preservation/stabilization of a native heart sac (pericardium), in order to subsequently enable a dimensionally stable cutting in the native state:

| | |
|---|---|
| 1 | Pericardium, e.g. from pigs, is freshly removed at the slaughterhouse and cooled for 2 h at 4°C in a suitable storage solution (e.g. EDTA/isopropanol, e.g. in aqueous solution). |
| ↓ | |
| 2 | After desired storage, the pericardial tissue is rinsed three times for 5 minutes in NaCl (0.9%), e.g., aqueous solution. |
| ↓ | |
| 3 | The pericardial tissue is then prepared wet in NaCl (0.9 %), e.g. in aqueous solution: Mechanical removal of excess fat/connective tissue, followed by rough cutting. |
| ↓ | |
| 4 | Followed by rinsing with NaCl (0.9%), e.g. in aqueous solution, with gentle agitation. |
| ↓ | |
| 5 | The cut pericardial tissue (approximately 6 cm x 8 cm in area) is then rinsed in 100 ml glycerol solution (30% glycerol in water) for 15 minutes with gentle mechanical agitation. |
| ↓ | |
| 6 | Followed by rinsing of pericardial tissue (approximately 6 cm x 8 cm in area) in 100 ml polyethylene glycol solution (40% PEG200 in water) for 15 minutes with gentle mechanical agitation. |
| ↓ | |
| 7 | Optionally followed by rinsing of pericardial tissue (approximately 6 cm x 8 cm in area) in 100 ml polyethylene glycol solution (40% PEG400 in water) for 15 minutes with gentle mechanical agitation. |
| ↓ | |
| 8 | Stabilization of the pericardial tissue e.g. by drying in a climatic cabinet by reducing the relative humidity from 95% to 10% over 12 h at 40°C drying. |
| ↓ | |
| 9 | Remove the pericardial tissue stabilized and dried in this way and shape it by laser cutting. |
| ↓ | |
| 10 | Store or further process the cut tissue (e.g., using three-dimensional glutaraldehyde crosslinking). |

### Example D (not according to the claimed invention)

The tissue of example C can be decellularized between process step 4 and 5 to obtain decellularized native stabilized dried pericardium. For this purpose the following additional process steps must be carried out,
4a) Decellularization of the pericardial tissue in surfactin-deoxycholic acid solution (e.g. 100 ml 0.06% surfactin and 0.5% deoxycholic acid in 0.9% NaCl for 22 h at 37°C by changing of the solution after every 2 h)
4b) rinsing the pericardial tissue with NaCl (0.9%) preferably 6x for 10 minutes preferably with gentle agitation
4c) Decellularization the pericardial tissue in DNase solution or DNase and cucumis melo solution the cucumis melo is obtained from green coffee bean (GCB): e.g. for 2d at 37°C
4d) rinsing the decellularized pericardial tissue with NaCl (0.9%) with TRIS (pH 11) e.g. 4x for 10 minutes under gentle agitation
4e) rinsing the decellularized pericardial tissue with ethanol solution e.g in 100 ml 70% ethanol solution at 37°C for 15 minutes
4f) preferably rinsing the decellularized pericardial tissue with NaCl (0.9%) e.g. 6x for 10 minutes under gentle agitation

### Example F (not according to the claimed invention)

Exemplary manufacturing process 700 may begin with step 701, in which step a pericardial tissue is selected as the biological tissue. This can come directly from a pig, which is obtained as part of the slaughter process and can be stored at a sufficiently low temperature of 1-6°C, preferably at 4°C for a period of 1h - 4h, preferably for 2h, in a storage solution (e.g. EDTA (ethylene diamine tetraacetic acid)/ISO, etc.) until further processing.

As a subsequent step (step 702), it is possible to rinse the biological material several times (e.g., twice, three times, four times, five times, etc.), preferably three times, in a saline solution (with a concentration of 0.5%-1.5%, preferably 0.9%). Each rinsing process can take e.g. 3 minutes, 4 minutes, 5 minutes, 6 minutes, 7 minutes, etc., but preferably 3 minutes. Any value between these values may also be possible within aspects of the second aspect of the invention.

In step 703, the biological tissue may be prepared (e.g., wet in NaCl at a concentration of, e.g., 0.9%) for subsequent process steps. This may include removal of fatty and/or connective tissue, and rough cutting of the biological tissue into the required shape.

In step 704, rinsing the biological tissue again in NaCl (with a preferred concentration of 0.9% may be performed) with gentle agitation. It should be noted that the rinsing process is not limited to NaCl and other suitable rinsing solutions may be used.

This may be followed, in step 705, by a rinsing process of the biological material, which has preferably been cut into a basic rectangular shape (e.g., 6 cm x 8 cm), in preferably 100 ml of glycerol solution (20-40% glycerol (preferably 30%) in ultrapure water) under gentle agitation for 5-20 minutes, preferably for 15 minutes.

In step 706, another rinsing step of the biological tissue in 100 ml of polyethylene glycol solution (10-50%, preferably 40%, PEG200 in ultrapure water) may follow. The rinsing time, under gentle agitation may be 10-25 minutes, preferably 15 minutes.

In step 707, another rinsing step may follow, in which the biological material may be rinsed in 100 ml of polyethylene glycol solution (10-50%, preferably 40%, PEG400 in ultrapure water). The rinsing time, under gentle agitation may be 10-25 minutes, preferably 15 minutes.

In step 708, the biological tissue can be dried in the climatic chamber by reducing the relative humidity from, for example, 90-98%, preferably from 95%, to 5-15%, preferably 10%, in 5-15 hours, preferably 12h, at 30-50°C, preferably at 40°C.

In step 709, the biological tissue 23 thus stabilized and dried can be removed and laser cut into the desired shape.

In step 710, the dried tissue can be further stored until it is used.

### Example G (not according to the claimed invention)

Example G describes an exemplary process 800 for producing native dried pericardium, which can be performed between process steps 704 and 705 of example F.

Step 801 comprises decellularizing biological tissue 23 in 100ml in, for example, surfactin-deoxycholic acid solution, for example comprising 0.06% surfactin and 0.5% deoxycholic acid in 0.9% NaCl. This process can be performed over a period of 15-30 hours, preferably for 22 hours at a temperature of 30-40°C, preferably at 37°C. Further, a change of solution can be performed after 1 h, 2 h, 3 h, 4 h, etc., preferably after 2 h.

In step 802, rinsing of the biological tissue 23 in 100 ml of NaCl (e.g., 0.5-1.5%, preferably 0.9%) can be performed with gentle agitation. The rinsing process may be repeated for 2-10 times (preferably six times), and each rinsing process may be performed for 5-15 minutes, preferably 10 minutes.

In step 803, decellularization in DNase solution may follow. This can be performed for 1-3 hours (preferably 2 hours) at 30-40°C, preferably at 37°C. Optionally, step 803 can be performed in the presence of a solution of DNase and GCB.

In step 804, rinsing of the biological tissue 23 in 100 ml of NaCl (e.g., 0.5-1.5%, preferably 0.9%) can be performed with gentle agitation. Additionally, tris(hydroxymethyl)aminomethane (TRIS), e.g. at pH 11, may be added to the NaCl solution.

Step 805 may be followed by rinsing the biological tissue 23 for one to five times (preferably once) for 5-20 minutes (preferably 15 minutes) in 100 ml of ethanol (60-80%, preferably 70%) at 30-40°C, preferably at 37°C.

The tissue sheets obtained in examples 1 to 5 can then be formed into a tube having an overlapping area of the tissue and be either crosslinked by using a glutaraldehyde solution (e.g. 0.5 vol% or wt%) and/or the overlapping area can be sutured (e.g. by using a polymer tread). Preferably sutureless tissue tubes (e.g. pericardial tissue tubes) are prepared, preferably being transparent and/ or having a thickness of less than 100 µm.

### Example H (not according to the claimed invention)

An exemplary process 900 shown Example H describes the crosslinking of a collagen-containing tissue e.g. pericardial tissue under pressure.

In step 901, this may involve cutting natively dried pericardium into a geometric shape suitable for the stent 1 using a CO2 laser.

In step 902, the shaping of a substantially cylindrical arrangement of the biological tissue may be performed.

In step 903, wrinkle-free placement of biological tissue and permeable material layer 5 (e.g., polyester fabric) on stent 1 and insertion of permeable material layer 5 in overlapping areas 4 (junctions of biological tissue 23 with itself) may occur.

In step 904, assembly into the rigid outer mold (e.g., counter element 21) may be performed. Further, the pressure generating means 22 (e.g., a dilatable tube) may be inserted into the biological tissue 23.

In step 905, the pressure generating means may be dilated, e.g., by applying a pressure of 5-15 bar, preferably 10 bar.

In step 906, the crosslinking form thus built up can be introduced into DPBS solution.

In step 907, the pressure can be reduced to 0.5-2 bar, preferably 1 bar, and after 1-5 minutes, preferably after 1 minute, it can be increased again to e.g. 10 bar.

In step 908, glutaraldehyde (with a concentration of 0.2-1%, preferably of 0.5%) can be added and the biological tissue 23 can thus be crosslinked for a period of 20-30 hours, preferably for 24 hours (at preferably constant pressure).

In step 909, the pressure can be released and the biological tissue 23, rinsed three to five times, preferably three times, for 1-10 minutes, preferably for 5 minutes, in 100ml Dulbecco's phosphatebuffered saline (DPBS) with gentle agitation. Stent 1 can then be removed with the biological sheath 3 applied and the permeable material layer 5 (e.g., the polyester fabric) removed.

In step 910 the thus obtained covered stent can be freely post-crosslinked in 0.1-1%, preferably in 0.5%, glutaraldehyde for 12 days, preferably changing the solution used every three days. According to step 911, drying of the encased stent 1 can subsequently be performed at least partially according to process 700.

In step 912, crimping of the produced biological sheath 3 onto a balloon catheter can take place. The product thus obtained can be packaged and finally sterilized in, for example, ETO (ethylene oxide).

### Example I

To ensure wrinkle-free crosslinking of pericardial tissue, the pericardium may be stretched on a plastic frame. Alternatively, the use of at least one permeable material layer disclosed herein, e.g., made of a polyester mesh, enables wrinkle-free stabilization.

For this purpose, according to the present embodiment, pericardial tissue is placed between the permeable material layers after mechanical preparation (e.g. removal of excess tissue, in particular fatty tissue, and cutting) and is pressed while drying the tissue. A transparent pericardium results directly from this method step. In this variant, the technical fabric serves as a permeable material, but not primarily for a two-dimensional water drainage option in the sense of drainage, but rather for shape stabilization.

Several permeable material layers, e.g. of technical tissue, e.g. three, four, five, six or more, can also be arranged and connected on top of each other in such a way that two adjacent layers each form a type of receiving pocket for a pericardial tissue.

The use of permeable material layers made of technical fabric enables water present in the pericardial tissue to be removed with comparatively low pressure, and on the other hand it ensures sufficient removal of the tissue water replacing material.

The compression of the pressure compensation layer under the effect of pressure between the rigid counterforms, results in a pressure load on the pericardial tissue. The water present in the pericardial tissue escapes via the layers of the technical fabric already during the assembly of the device. Depending on the applied pressure load, thickness-reduced tissues can be obtained.

The pressure compensation layer (e.g. a polyurethan foam or a silicone) is characterized by the parameters: Compression hardness, density, material composition, material thickness and, if necessary, permeability. Since in some embodiments the pressure is regulated by a stepless reduction of the distance between two rigid counter-forms, for example by suitable connecting means in combination with steplessly adjustable retaining means, the pressure compensation layer is of particular importance with regard to the transmission of pressure to the tissue to be treated. Compression hardness, bulk density, thickness and type of material used directly influence the maximum achievable pressure at a defined and desired distance between the counterforms. The higher the compression hardness and bulk density are selected, and the thicker the material, the higher the pressure built up on the tissue.

Hydrophobic materials therefore appear less suitable for the pressure compensation layer with this context. Nevertheless, hydrophilic pressure compensation layers are preferred.

In a particularly preferred embodiment the pressure compensation layer comprises the following set of parameters, which is very suitable for the formation of thickness-reduced tissue, in particular ultracompact tissue:
Compression hardness: 60 kPa
density: 40 kg/m³
Thickness: 3 cm
Material: polyurethane foam

The applied compression pressure/pressure load is another important influencing parameter. In general, at lower pressures, this results directly in higher tissue thickness. Higher pressures in turn reduce the tissue thickness; but are quasi ineffective after a certain point of thickness reduction, but still cause a stiffening of the tissue, which can be illustrated by bending behavior measurements.

Preferred is a compression pressure/load in the range of 0.002 kg/cm² to 0.15 kg/cm².

Depending on the requirements of the tissue to be treated and for the particular application, different compression pressures/pressure loads may be preferred.

One problem of the permeable material layer(s) of the technical fabric disclosed herein, for example, is to ensure a sufficient surface area for the exchange of liquids, in particular of the tissue water and the crosslinking solution, during crosslinking under pressure. This includes, on the one hand, the drainage of water from the biological tissue during compression/pressure loading (drainage function) and, on the other hand, the removal of the hydroscopic exchange medium from the tissue to be treated. Characteristic parameters are therefore essentially the surface properties, permeability and stiffness of the permeable material layer(s), e.g. of the technical fabric used here.

Due to the direct contact of the permeable material layer(s) with the tissue to be press, special requirements must be placed on the surface of the technical tissue, for example, such as the absence of detaching particles or also the structuring of the material layers themselves, since these may be imprinted in the (biological) tissue during pressing.

Preferred are polyester meshes with 100 to 180 threads/cm. The mesh size ranges from 10 µm to 60 µm, with a thickness of the technical fabric of about 40 µm to 70 µm being suitable. Larger mesh sizes also lead to a sufficient exchange of fluids, but the imprinted structure on the surface of the biological tissue may then even be visible to the naked eye.

Nevertheless, in another embodiment these properties of the imprinted surfaces of the permeable material layers can also be used in a positive sense. Namely, whenever one wishes to imprint a technically functional surface on the tissue to be treated, such as, for example, a roughening of the tissue surface or specific depressions in the tissue surface, etc.

### Example J

The individual concrete method steps for producing a planar porcine pericardial tissue can be summarized as follows:
(i) A pericardium, e.g. from a pig, is freshly collected at the slaughterhouse and stored for 2 h at 4°C in a suitable storage solution, e.g. EDTA/isopropanol or saline;
(ii) The pericardial tissue is rinsed three times for 5 min in saline (0.9%);
(iii) The pericardial tissue is prepared wet in saline (0.9%): Removal of fat/connective tissue and this is followed by gross cutting to approximately 12 cm x 8 cm;
(iv) Followed by irrigation in physiological saline with gentle mechanical agitation;
v) Provision of a first rigid counterform comprising holes - lower counterform;
vi) fitting the first rigid counterform with a suitable connecting means, e.g. screws;
vii) Placement of a first permeable material (technical fabric) centrally on the first rigid counterform as a support surface without folds;
viii) Central wrinkle-free support of the pericardial tissue on the first permeable material, followed by a smoothing out of any air bubbles present in the pericardial tissue;
ix) Central wrinkle-free overlay of a second permeable material of technical tissue on the pericardial tissue;
x) central overlay of a pressure compensation layer of polyurethane foam on the second permeable material;
xi) Precisely fitting and form-fitting support of a second rigid counterform with perforations and comprising holes;
xii) connecting the two counterforms via the screws and fixing the counterforms by means of a suitable continuously adjustable retaining means, such as, for example, one or more nuts;
xiii) adjustment of a desired pressure load (application of force) between the counterforms via a suitable regulation of the distance between the two plates; for example, via a looser or tighter screwing of both plates. This means that the pressure load is controlled as a function of the plate spacing, which can be implemented mechanically, hydraulically or via a suitable pneumatic system, for example;
xiv) placing the device/crosslinking unit in a suitable receptacle/container - e.g. substantially vertically or substantially horizontally;
xv) Filling the container/receptacle with a sufficient amount of 0.5% glutaraldehyde solution such that the device/crosslinking unit is completely covered with the crosslinking solution;
xvi) Followed by chemical crosslinking for 2 days at a temperature of 37°C;
xvii) Storage of the final processed pericardial tissue in glutaraldehyde solution or followed by any further processing.

The planar sheet can be rolled up to form a tissue tube and overlapping area of the tissue can be sutured e.g. with a (polymer) thread.

### Example K

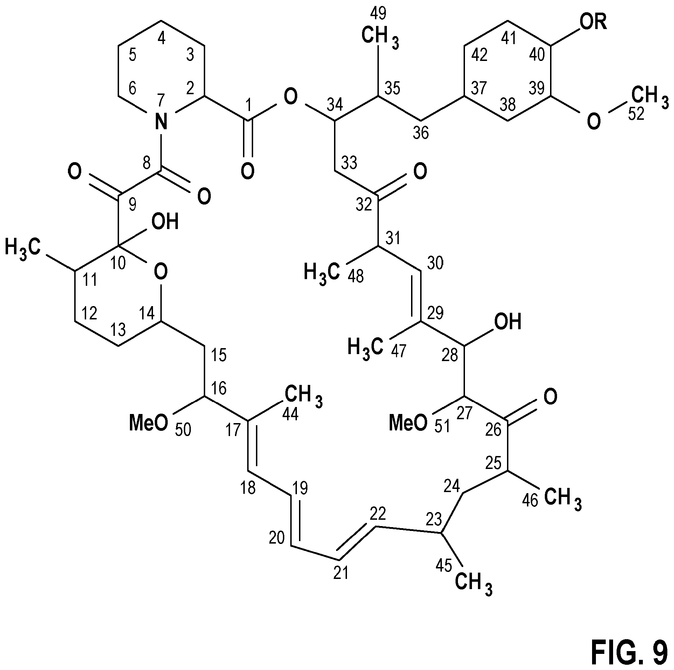

The (dried or stabilized dried) tissue tube (e.g. pericardial tissue tube) may be further immersed in a solution containing at least one drug e.g. at least one anti-inflammatory drug and/or at least one immunosuppressant drug and/or at least one mTOR inhibitor like a sirolimus or derivative thereof (e.g. as described in Fig. 9).

### Example L (not according to the claimed invention)

A decellularized biological tissue / extracellular matrix can be solubilized. The solubilizing the extracellular matrix of the biological tissue includes one of:
- chemically solubilizing the extracellular matrix, e.g. by acidic digestion. Acidic digestion can be performed e.g. in 1 N HCl for 12 h with subsequent neutralization using NaOH, centrifugation to obtain the constituents of solubilized extracellular matrix and resuspension of the pellet in 0.9%w/v NaCl to obtain a concentrated suspension;
- enzymatically solubilizing the extracellular matrix, e.g. by disintegration with collagenase, Enzymatic disintegration can be conducted e.g. by using 4 U/ml collagenase type IV from clostridium histolyticum, (Biochrom, Catalog# C4-28; other types of proteinases may also be used, especially unspecific proteinases like e.g. 'proteinase K') for 12 h in a 50 mM TES buffer (AppliChem, Catalog# A1084) containing 10 mM Ca, centrifugation to obtain the constituents of solubilized extracellular matrix and resuspension of the pellet in 0.9%w/v NaCl to obtain a concentrated suspension;
- wet mechanically solubilizing the extracellular matrix, e.g. by mechanical homogenization in solution: e.g. grinding of the extracellular matrix into a suspension using a homogenizer (Bertin, Precellys Evolution) and ceramic pellets (Bertin, Catalog# CK14) in 0.9%w/v NaCl; in doing so the tissue is cooled during homogenization;
- dry mechanically solubilizing the extracellular matrix in the solid state, e.g. by homogenization of the frozen extracellular matrix: e.g. by liquid Nitrogen in the form of comminuting the frozen extracellular matrix by grinding (Bertin, Precellys Evolution with ceramic pellets (Bertin, Catalog# CK14) or manually pestling, thawing of the comminuted extracellular matrix and resuspension in 0.9%w/v NaCl.

The solubilizing by enzymatic disintegration of pericardium is best performed by use of collagenase type IV since pericardium includes a high content of dominant collagen fibers which are well digested by collagenase type IV. For other types of tissue different proteinases may be better suited such as the rather unspecific proteinase K.

The preparation of a tissue tube may then be carried out as described in the examples 1 to 60. Briefly, solubilized extracellular matrix /decellularized biological tissue, preferably decellularized and solubilized pericardial tissue is placed between an inner hollow cylinder and an outer tube. The inner cylinder has an outer diameter. The outer tube has an inner diameter being larger than the outer diameter of the inner cylinder.

The outer tube and/or the inner cylinder or the inner tube is/are made of a permeable and/or porous material. The hollow cylinder is preferably permeable for tissue water, the water replacing material (Glycerin, and or PEG) and/or a crosslinking agent (glutaraldehyde solution). Due to this tissue water can escape from the tissue e.g. when pressing the tissue and/or a water replacing material (Glycerin, and or PEG) and/or a crosslinking agent (glutaraldehyde solution) can pass through the pores of the permeable and/or porous material and reach the biological tissue to be treated.

The solubilized extracellular matrix /decellularized biological tissue, preferably decellularized and solubilized pericardial tissue, is then crosslinked preferably by using a glutaraldehyde solution (e.g. 0.5wt%)

One or more pressure compensation layers are placed between the one or more native biological tissue(s) or the one or more decellularized biological tissue(s) and the outer tube.

Optionally the solubilized extracellular matrix /decellularized biological tissue, preferably decellularized and solubilized pericardial tissue, is structurally stabilizing with a tissue water replacing material before or after the chemical crosslinking and is then optionally dried.

Native biological tissue is incubated in stabilizers in order to maintain the mechanical flexibility. The biological tissue pre-treated in this way is then mechanically pressed in a compression mold in technical fabrics and open-cell foams for drainage. This densifies the fiber structure of the fabric, forms physical bonds between the protein fibers, removes excess stabilizers and, in particular, eliminates voids between the protein fibers, resulting in the required optical transparency of the material.

### Description of the figures

Fig. 1 shows in schematic cross-section the structure/arrangement of various material layers in a pressing unit for pressure loading (the arrows represent the pressure loading schematically) suitable for the methods of the first aspect of the invention. As can be seen in Fig. 1, the drying of the (pericardial) tissue (8) takes place while it is arranged/placed in the pressing unit consisting of two rigid but optionally perforated counterforms (10, 11), a polyurethane foam (e.g. 10 - 30 mm in height) as an additional pressure compensation layer (12), and two permeable material layers of e.g. technical fabric (7a, 7b with drainage function; e.g. 50 µm polyester with 40 µm meshes). The biological tissue is placed between two permeable material layers. Furthermore, the flexibility of the pericardial tissue can be modified in addition to the final thickness by adjusting the plate spacing (10, 11).

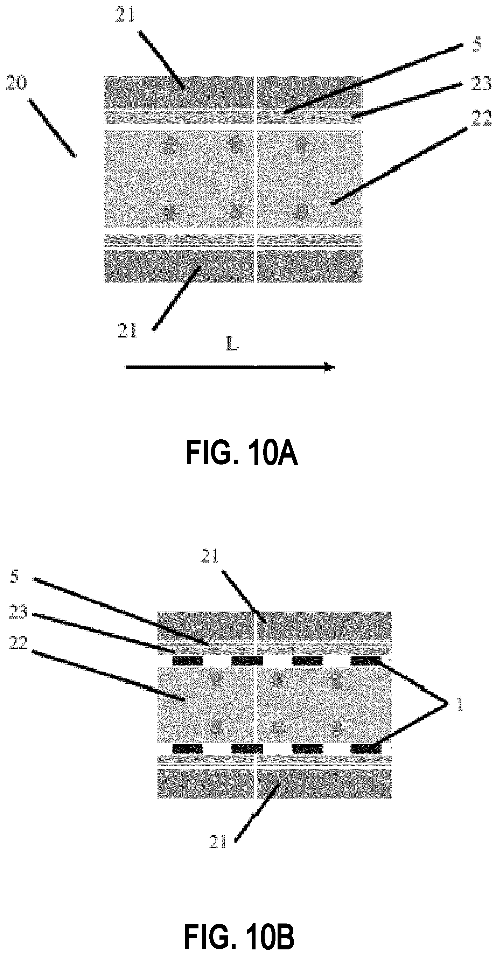

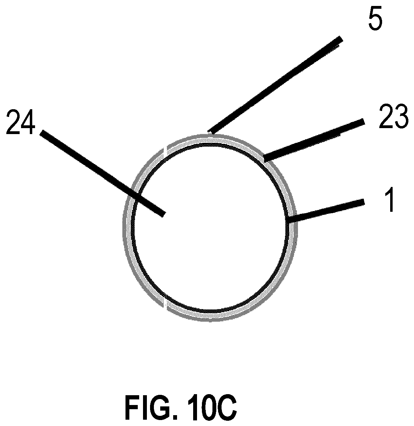

Depending on the starting tissue used, different end thicknesses can be achieved. For example, for porcine pericardial tissue, the use of two permeable material layers (7a, 7b), e.g. of technical tissue, results in a thickness reduction of 50% already at a pressure load of about 0.1 kg/cm². Depending on the starting tissue, however, a greater thickness reduction can also be achieved by increasing the pressing force (see Fig. 9 above, Fig. 10), with complete fiber compaction representing a lower limit of the final thickness. In the case of porcine pericardium, for example, this is about 20 µm.

According to the first aspect of the invention, the technical fabric as a permeable material layer has essentially two functions: (i) Draining the water from the tissue when the external pressure is applied; (ii) Improving the accessibility of a tissue water replacing material to the tissue during the pressing. So not only a drainage function, because of the fiber structure, the technical fabric, such as mesh, additionally "conducts" liquid from one position to another.

The use of the permeable material layers (7a, 7b), e.g. made of technical fabric, enables through its permeable properties on the one hand that water present in the pericardial tissue can be removed with comparatively low pressure (in the sense of drainage), and on the other hand ensures sufficient accessibility of the tissue water replacing material solution to the pericardial tissue (8). This can optionally be promoted by the fact that the rigid counterforms (10, 11) are either both or only one of them, preferably the upper one (1), additionally perforated and also facilitate access to the pericardial tissue (8).

By compressing the e.g. polyurethane foam as a pressure compensation layer (12) under an applied pressure load between the rigid counterforms (10, 11), a desired and continuously adjustable pressure is exerted on the pericardial tissue (8). The water present in the pericardial tissue escapes via the layers of the technical fabric as permeable material layers (7a, 7b) already during the assembly of the previously described pressing unit.

The use of the pressure compensation layer (12), e.g. polyurethane foam or a silicone mat, serves to transfer the force of the applied pressure of the rigid counterforms (10, 11) and ensures compensation of the natural inhomogeneity of the tissue (8), thus avoiding local stress peaks. The resulting tissue thus exhibits improved thickness homogeneity. Furthermore, in addition, such a pressure compensation layer promotes the wetting of the pericardial tissue (8). By continuously reducing the plate spacing of the counterforms (10, 11) and the associated compression of the foam (12), the final thickness of the pericardial tissue (8) can be specifically adjusted to the requirements of a subsequent application, in particular for a medical application as an implant.

Figure 2: shows another embodiment of compression mold/pressing unit for stabilized drying of biological tissue (e.g. pericardium) under external pressure: Biological tissue between two rigid outer molds/counterforms, both of which are porous or have holes. Optionally, an open-pored polyurethane foam can also be inserted on one or both sides.

The pressing unit, having from bottom to top the following parts:
- first (non-porous, porous or perforated) counterform (10), e.g. porous ceramic counterform,
- first permeable material layer (7a), e.g. a polyester mesh
- optionally the biological tissue (8) to be treated,
- second permeable material layer (7b), e.g. a polyester mesh
- second (non-porous, porous or perforated) counterform (11), e.g. porous ceramic counterform.

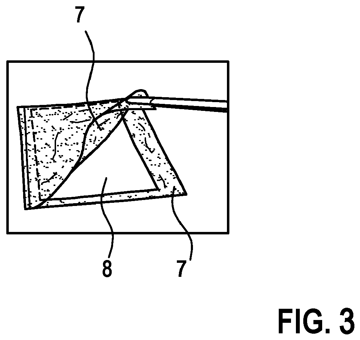

Fig. 3 shows a (structurally stabilized) tissue (8) being placed in a permeable material layer (7), which means the permeable material layer (7) is folded on the tissue (8) or wraped around the tissue (8).

Fig. 4A-H schematically show the individual method steps for setting up the pressing unit described in Fig. 1 for producing a planar pericardial tissue. Fig. 4A shows a lower rigid counterform (11) with several holes (13) as an initial stage. Fig. 4B shows the lower counterform (11), whereby all holes (13) are equipped with a continuously adjustable connecting means, e.g. a screw (14), in such a way that the screws themselves embody quasi joining rails, by means of which the above counterform (10) can be connected to the lower counterform (10), e.g. with an accurate fit and a positive fit. In Fig. 4C, a first layer of permeable material (7b), e.g. of technical fabric, is placed in the center of the device without folds. In Fig. 4D, the pericardial tissue (8) is placed on top of the first layer of permeable material (7b), e.g. of technical tissue, without folds in the center. In Fig. 4E, a second layer of permeable material (7a), e.g. of technical fabric, is placed without folds centrally in the device over the pericardial tissue (8) and thus also over the first permeable material layer (7b). In Fig. 4F, a pressure compensation layer of polyurethane foam (12) is placed centrally in the device, and thus comes to rest above the second permeable material layer (7a), above the pericardial tissue (8), and above the first permeable material layer (7b). In Fig. 4G, the upper rigid counterform (10) with perforations (10a) is precisely and positively joined over the holes also present in (10) and the screws (14). In Fig. 4H, the screws (14) are each connected with a nut (14a) as a connecting means in such a way that the screw connection via the nuts (14a) allows a pressure load to be set on all material layers and the tissue in the device and also to be released again. In other words, the distance between the rigid counterforms (10, 11) can be selectively and continuously adjusted via the connecting means (14, 14a) to suit the situation, depending on the tissue to be treated.

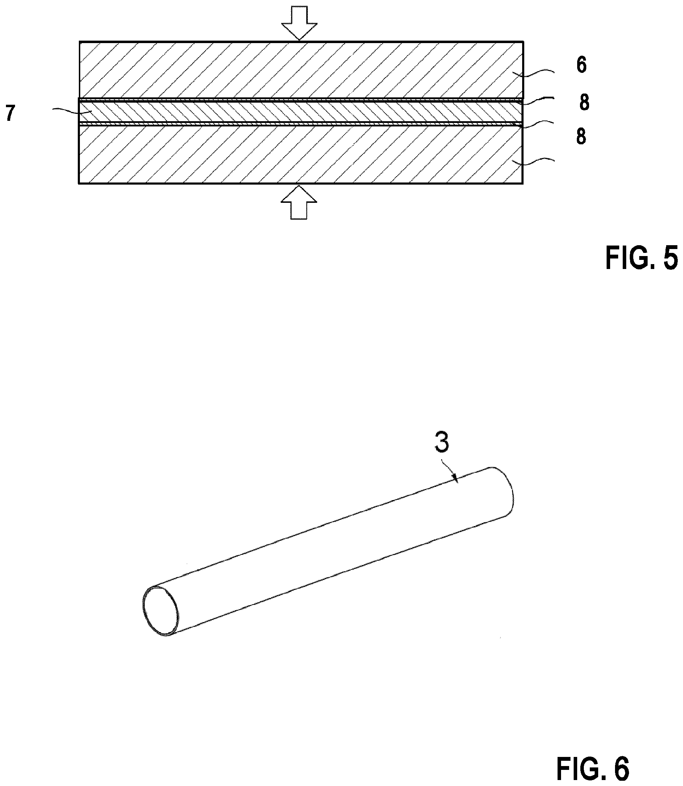

Fig. 5 shows in schematic cross-section the arrangement of a biological tissue in a pressing unit for pressure loading (the arrows represent the pressure loading schematically) suitable for the methods of the invention. As can be seen in Fig. 5, the cross-linking of the (pericardial) tissue 8 takes place while it is arranged/placed in the pressing unit comprising an outer hollow cylinder/outer tube 6 and an inner (solid or hollow) cylinder 7. The outer tube has an inner diameter being larger than the outer diameter of the inner cylinder or the inner tube. The outer tube may be made of more than on piece, e.g. 2 halfs.

The outer tube and/or the inner (solid) cylinder or the inner (hollow) tube is/are made of a permeable/porous material.

An optional polyurethane foam (e.g. 10 - 30 mm in height) placed between the outer tube and the tissue and/or the inner tube and the tissue can act as a pressure compensation layer (not shown),

The permeable/porous material may be a polymer fabric (with drainage function; e.g. a 50 µm polyester with a mesh size or pore size of 40 µm). The biological tissue is placed between the outer hollow cylinder/outer tube 6 and the inner (solid) cylinder or the inner (hollow) tube 7.

Depending on the starting tissue used, different end thicknesses can be achieved. For example, for porcine pericardial tissue, the use of a polymer fabric, e.g. polyester, results in a thickness reduction of 50% already at a pressure load of about 0.1 kg/cm². Depending on the starting tissue, however, a greater thickness reduction can also be achieved by increasing the pressing force, with complete fiber compaction representing a lower limit of the final thickness. In the case of porcine pericardium, for example, this is about 20 µm.

According to the invention, the technical fabric as a permeable material layer has essentially two functions: (i) Draining the water from the tissue when the external pressure is applied; (ii) Improving the accessibility of the cross-linking agent to the tissue during the pressing. So not only a drainage function, because of the fiber structure, the technical fabric, such as mesh, additionally "conducts" liquid from one position to another.

The use of the permeable/porous material, e.g. made of a porius polymer fabric, enables through its permeable properties on the one hand that water present in the pericardial tissue can be removed with comparatively low pressure (in the sense of drainage), and on the other hand ensures sufficient accessibility of the cross-linking agent solution to the pericardial tissue (8).

By compressing the e.g. polyurethane foam as a pressure compensation layer under an applied pressure load between the rigid counterforms, a desired and continuously adjustable pressure is exerted on the pericardial tissue (8). The water present in the pericardial tissue escapes via the layers of the technical fabric as permeable material layers (7a, 7b) already during the assembly of the previously described pressing unit.

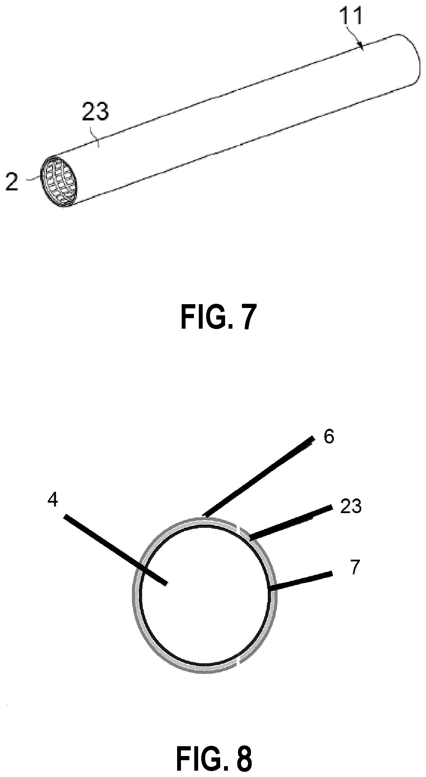

The use of the pressure compensation layer (12), e.g. polyurethane foam or a silicone mat, serves to transfer the force of the applied pressure of the rigid counterforms (10, 11) and ensures compensation of the natural inhomogeneity of the tissue (8), thus avoiding local stress peaks. The resulting tissue thus exhibits improved thickness homogeneity (see Fig. 8). Furthermore, in addition, such a pressure compensation layer promotes the wetting of the pericardial tissue (8). By continuously reducing the plate spacing of the counterforms (10, 11) and the associated compression of the foam (12), the final thickness of the pericardial tissue (8) can be specifically adjusted to the requirements of a subsequent application, in particular for a medical application as an implant.

Fig. 6 shows a biological tissue tube 3 (having no sutures and no support structure). The tissue tube 3 can be a collagenous tissue tube, preferably a pericardial tissue tube. The tissue may be autologous tissue. The tissue tube 3 can be used as bypass or stent (with or without support structure). The tissue 3 tube may comprise drug molecules, e.g. anti-inflammatory drugs or immunosuppressant drugs or mTOR inhibitors like sirolimus or derivatives thereof.

Fig. 7 shows a covered stent or stent graft 11 having a support structure 2 and a biological tissue 23 covering the support structure 2. The biological tissue covering can be a collagenous tissue tube, preferably a pericardial tissue tube. Here the biological tissue covering is only on the outer side of the support structure, however the biological tissue covering can also cover the inner and outer side of the support structure. Alternatively, the biological tissue covering material can also cover all side as and also the edges of the stent. The support structure may me made of a metal, a metal alloy or a polymer, preferably containing Nickel (Ni) and Titanium (Ti), e.g. a NiTi or NiTinol. The support structure shown has a tubular mesh structure, however hollow cylindrical support structures may be possible as well. The support structure can be made of a self-expandable or self-expanding material.

Fig. 8 shows the preparation of a tissue tube, wherein the one or more biological tissue (s), preferably pericardial tissue is placed between an inner cylinder and an outer tube 6. Here the inner cylinder 7 has a hollow space 4, thus the cylinder is a hollow cylinder (also called inner tube). The inner cylinder 7 has an outer diameter. The outer tube 6 has an inner diameter being larger than the outer diameter of the inner cylinder 7.

The outer tube and/or the inner cylinder or the inner tube is/are made of a permeable and/or porous material. The hollow cylinder is preferably permeable for tissue water, the water replacing material (Glycerin, and or PEG) and/or a crosslinking agent (glutaraldehyde solution). Due to this tissue water can escape from the tissue e.g. when pressing the tissue and/or a water replacing material (Glycerin, and or PEG) can pass through the pores of the permeable and/or porous material and reach the biological tissue to be treated.

One or more pressure compensation layers are placed between the one or more native biological tissue(s) or the one or more decellularized biological tissue(s) and the outer tube.

The biological tissue may be solubilized biological tissue or biological tissue being at least 1cm²

Fig. 9 shows a sirolimus derivate, where R is C(O)-(CH2)n-X, n is 0, 1 or 2, X is a cyclic hydrocarbon having 3-8 carbons, optionally containing one or more unsaturated bonds, one or more linear-chain or cyclic carbon atoms may contain an OH or halide substitution, and the rapamycin parent structure may contain substitutions at other positions.

Exemplary R groups include:

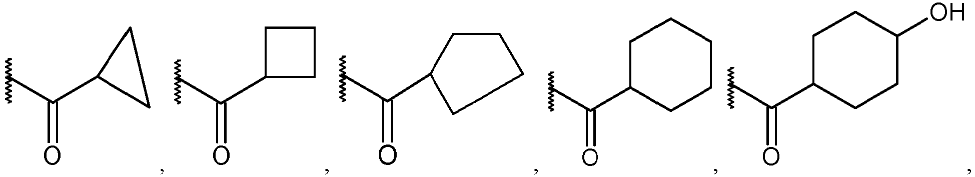

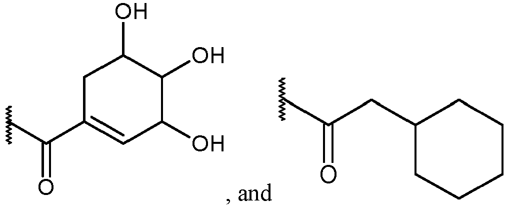

This sirolimus derivative can be incorporated into the tissue tube, preferably the pericardial tissue tube, or into a covered stent comprising said tissue tube or the tissue tube, preferably the pericardial tissue tube, can be covered with this sirolimus derivative.

Figures 10A to 10C show exemplary embodiments of a device for manufacturing a tissue tube.

Fig. 10A shows a possible embodiment for a device 20 for manufacturing a tissue tube 3 in a cross-sectional view shown along a longitudinal extension.

The device 20 comprises an e.g. one- or two-part counter element 21, which is preferably provided with a cylindrical receiving means for receiving therein the preferably cylindrically configured pressure generating means 22.

Optionally, it is possible, e.g. for longer lengths of the stent 1, to provide the counter element 21 with holes, to design the inner surface of the counter element 21 with fine superficial channels or with inherent porosity (e.g. ceramic). This may allow for additional convective and diffusive mass transfer.

The counter element 21 may also have surface structures that extend into open regions of the stent 1 (Fig. 10B). This may allow geometric adaptation of the biological tissue 23 into the stent 1, providing additional support of the biological tissue 23 that goes beyond mere embedding of the stent (esp. the stent strut) into the planar biological tissue 23.

The pressure generating means 22 (e.g., a hydraulically and/or pneumatically dilatable balloon catheter) may be provided to dilate along a radial direction (perpendicular to the longitudinal direction L). A biological tissue 23 to be crosslinked may further be provided around the pressure generating means 22, which may preferably be provided as a substantially cylindrical arrangement and may be pressed against the counter elements 21 by the dilatation of the pressure generating means 22 (exemplarily represented by the direction of the arrows shown in Fig. 10A).

Between the biological tissue 23 as well as the counter elements 21, a pressure compensation layer 5 is introduced, which can be provided as described herein.

In this arrangement, cross-linking of the biological tissue 23 can be achieved.

Fig. 10B shows a second possible embodiment for a device 20 for manufacturing a biological tissue tube 3 in a cross-sectional view shown along a longitudinal extension.

The manufacturing device shown with reference to Fig. 10B is thereby based on the manufacturing device shown with reference to Fig. 10A, whereby a stent 1 can additionally be introduced between the pressure-generating means 22 and the biological tissue 23 (preferably from natively dried pericardium), thus making it possible to also carry out crosslinking of the biological tissue 23 onto the stent 1.

Fig 10C shows a third possible embodiment for a device 20 for producing a biological sheath 3 in a cross-sectional view of the device, perpendicular to its longitudinal extension. Instead of the pressure generating means 22 shown in Figs. 10A and 10B, a pin-like element 24 (e.g., a steel pin) may be introduced into the inner lumen of the stent 1 with respect to the device as shown in Fig. 10C.

By exerting pressure, by means of the pin-like element 24 in the direction U, the cylindrical arrangement (which may be configured as shown in Fig. 10A or 10B) can be pressed into a pressure compensation layer 25 (e.g., polymer foam, preferably polyurethane with a compression hardness of, for example, 60 kPa). The biological tissue 23 is preferably arranged in such a way that the parts to be crosslinked are at the bottom, in the direction U.

The pressure compensation layer 25 is provided such that it applies a counterforce against the application of pressure, such that an application of pressure to the portions of the biological tissue 23 to be crosslinked may be achieved.

The pressure compensation layer 25 may further be provided on a (rigid) plate 26, so as to provide a stable base for the application of pressure by the pin-like element 24.

It is further noted that device 20 according to Fig. 10C may also be used without stent 1. Furthermore, it is also possible to additionally insert the permeable material layer 5 between (overlapping) layers of the biological tissue 23, which are not to be crosslinked with each other.

In this context, it can be further noted that the elongation at break of pericardium crosslinked under tension in the plane of the biological tissue 23 or under external pressure is lower than the elongation at break of pericardium crosslinked freely under minimal external force. In this context, free crosslinking of the pericardium can be motivated without external pressure or internal mechanical tension on the collagen fibers during cross-linking. External pressure may be applied only at the point where stable connection of portions of the biological tissue 23 is required.

Using the fabrication device 20, as shown in Fig. 10C, the technical arrangement allows high mechanical pressure to be applied to the junction of the biological tissue 23 for tissue bonding, while in most of the biological sheath 3, crosslinking occurs with no or much lower mechanical pressure. A biological sheath 3 with an overall greater elongation at break can thus be obtained.

The previously discussed manufacturing devices may further have in common that in case of crosslinking under pressure, a sufficient exchange of fluids can be ensured, which can be supported in particular by the permeable material layer 5. This may be aided, on the one hand, by the removal of water from the biological tissue 23 during compression by the application of pressure.

Preferably, the permeable material layer 5 can be provided as a polyester screen mesh with 100-180 threads per cm. The mesh size of the fabric may range from 10-60µm, and a thickness of the biological tissue 23 may be preferred to be comparatively thin, with a thickness of about 40µm-70µm being advantageous.

In any of the aforementioned embodiments for the manufacturing device, DPBS (Dulbecco's Phosphate Buffered Saline Solution; pH 7.4) may be introduced into the device in the state of applying pressure to the biological tissue 23. Under pressure, stabilizers stored via the drainage can be replaced by DPBS, thus rehydrating biological tissue 23. The built-up pressure can also compress and reduce the thickness of the biological tissue 23. At the junctions of the biological tissue 23, the application of pressure can achieve a mechanically intimate connection between the parts of the biological tissue 23 by locally displacing the collagen fibers. The process of rehydration may optionally be assisted by an alternating pressure profile, which may support mass transfer by a pumping motion of the internal dilatable structure.

The crosslinking can be carried out under static pressure or preferably an alternating pressure profile, which can assist mass transfer by pumping motion of the internal dilatable structure. For example, crosslinking under pressure for 2 days at 37°C or for 4 days at room temperature have proven successful in experiments conducted by the applicant in previous series of experiments, although parameter constellations deviating from these can also provide a satisfactory result (such as crosslinking in a temperature range of 15-45°C over a period of 1-5 days).

The goal of crosslinking in the mold under radial pressure can be the dimensionally stable execution of the desired three-dimensional geometry of the biological tissue 23, the reduction of the material thickness of the biological tissue 23 to typically 30-70 micrometers, and the mechanically stable connection of contacting layers of the biological tissue 23.

Once this state has been reached, stent 1 with applied biological coating 3 can be removed from the device 20 and optionally completely crosslinked in further crosslinking steps outside the device 20 while constantly changing the crosslinking agent. By this additional post-fixation, the amount of not yet fixed free amino groups can be reduced with optimal accessibility of the crosslinking agent to the biological tissue 23. Thus, a final crosslinking state comparable to standard processed pericardial patches can be achieved. Further optional process steps, such as final crosslinking at elevated temperature or capping of unsaturated aldehyde groups, may also be possible in order to achieve improved properties of the biological tissue 23 with regard to in vivo calcification.

## Claims

1. Method for the preparation of a biodegradable tissue, wherein the method comprises or consists of the following steps:
a) providing one or more native biological tissue(s) or one or more decellularized biological tissue(s),
b) structurally stabilizing the one or more native biological tissue(s) or the one or more decellularized biological tissue(s) with a tissue water replacing material to obtain a structurally stabilized tissue,
c) providing at least one, preferably two, permeable material layer(s),
d) placing the structurally stabilized tissue on, in or between the permeable material layer(s);
e) providing one or more pressure compensation layers and covering the structurally stabilized tissue or the at least one, preferably two, permeable material layer(s), with the one or more pressure compensation layers;
f) drying the structurally stabilized tissue while applying a pressure to the structurally stabilized tissue to obtain a dried tissue,
g) optionally removing the dried tissue from the permeable material layer(s) and the optional pressure compensation layer(s);

2. The method according to claim 1, wherein the native biological tissue is a collagen containing biological tissue or the decellularized biological tissue is based on a collagen containing biological tissue.

3. The method according to claim 1, wherein the native biological tissue is dura mater or the decellularized biological tissue is based on dura mater.

4. The method according to any one of the preceding claims, wherein the at least one, preferably two, permeable material layer(s) comprise or consist of a polymer.

5. The method according to any one of the preceding claims, wherein the at least one, preferably two, permeable material layer(s) has/have a pore size in the range of 10 to 60 µm.

6. The method according to any one of the preceding claims, wherein the pressure compensation layer has a compression hardness in the range of 20 kPa to 80 kPa, preferably 30 kPa to 70 kPa, more preferably 40 kPa to 60 kPa, even more preferably 60 kPa.

7. Biodegradable tissue obtained by the method according to one of the preceding claims.

8. Biodegradable tissue according to claim 7 which is a biodegradable dura mater obtained by the method according to one of the claims 3 to 6, being optically transparent and/ or having a thickness of less than 80 µm, preferably between 60 and 20 µm.

9. Medical implant comprising the biodegradable tissue according to claims 7 or 8 or obtained by the method according to any one of the claims 1 to 6.

10. The medical implant according to claim 9, wherein the medical implant is selected from a covered stent or a stent graft.

11. The medical implant according to claim 9, wherein the medical implant is a cardiovascular implant, an endovascular prostheses, an endoprostheses, an esophageal implant, a bile duct implant, a dental implant, an orthopedic implant, a neurological implant, a microchip containing implant, a covered stent, a stent graft, a bone implant, a glucose sensor implant, a neurostimulator, a cochlear implant, an endoprostheses for closing persistent foramen ovale, an endoprostheses for closing an atrial septal defect, a left atrial appendage closure device, a pacemaker, a leadless pacemaker, a defibrillator, a prosthetic heart valve, a venous valve, a tooth implant.

12. Biodegradable biological tissue obtained by a method according to one of the claims 1 to 6 for use as a medical implant or for use in a medical implant.

13. Biodegradable biological tissue obtained by a method according to one of the claims 1 to 6 for use as a tissue patch.

14. Biodegradable dura mater obtained by a method according to one of the claims 3 to 6 for use as a tissue patch or for use as a tubular medical implant or for use of repairing dura mater defects.

15. Device for the preparation of biodegradable tissue according to a method of any one of claims 1 to 6 comprising:
- at least one pressure applying unit,
- one or more pressure compensation layer(s) being arranged in the pressure applying unit and
- one or more permeable material layer(s) being arranged in the pressure applying unit.

## Patentansprüche

1. Verfahren zur Herstellung eines biologisch abbaubaren Gewebes, wobei das Verfahren die folgenden Schritte umfasst oder aus diesen besteht:
a) Bereitstellen eines oder mehrerer nativer biologischer Gewebe oder eines oder mehrerer dezellularisierter biologischer Gewebe,
b) strukturelles Stabilisieren des einen oder der mehreren nativen biologischen Gewebe oder des einen oder der mehreren dezellularisierten biologischen Gewebe mit einem Gewebewasser-Ersatzmaterial, um ein strukturell stabilisiertes Gewebe zu erhalten,
c) Bereitstellen von mindestens einer, vorzugsweise zwei, Schicht(en) aus einem permeablen Material,
d) Anordnen des strukturell stabilisierten Gewebes auf, in oder zwischen der/den Schicht(en) aus einem permeablen Material;
e) Bereitstellen einer oder mehrerer Druckausgleichsschichten und Abdecken des strukturell stabilisierten Gewebes oder der mindestens einen, vorzugsweise zwei, Schicht(en) aus einem permeablen Material mit der/den Druckausgleichsschicht(en);
f) Trocknen des strukturell stabilisierten Gewebes unter Ausübung eines Drucks auf das strukturell stabilisierte Gewebe, um ein getrocknetes Gewebe zu erhalten,
g) optional Entfernen des getrockneten Gewebes von der/den Schicht(en) aus einem permeablen Material und der/den optionalen Druckausgleichsschicht(en).

2. Verfahren nach Anspruch 1, wobei das native biologische Gewebe ein kollagenhaltiges biologisches Gewebe ist oder das dezellularisierte biologische Gewebe auf einem kollagenhaltigen biologischen Gewebe basiert.

3. Verfahren nach Anspruch 1, wobei das native biologische Gewebe die Dura mater ist oder das dezellularisierte biologische Gewebe auf Dura mater basiert.

4. Verfahren nach einem der vorstehenden Ansprüche, wobei die mindestens eine, vorzugsweise zwei, Schicht(en) aus einem permeablen Material ein Polymer umfasst/umfassen oder daraus besteht/bestehen.

5. Verfahren nach einem der vorstehenden Ansprüche, wobei die mindestens eine, vorzugsweise zwei, Schicht(en) aus einem permeablen Material eine Porengröße im Bereich von 10 bis 60 µm aufweist/aufweisen.

6. Verfahren nach einem der vorstehenden Ansprüche, wobei die Druckausgleichsschicht eine Druckhärte im Bereich von 20 kPa bis 80 kPa, vorzugsweise 30 kPa bis 70 kPa, noch bevorzugter 40 kPa bis 60 kPa, am meisten bevorzugt 60 kPa aufweist.

7. Biologisch abbaubares Gewebe, erhalten durch das Verfahren gemäß einem der vorstehenden Ansprüche.

8. Biologisch abbaubares Gewebe gemäß Anspruch 7, bei dem es sich um biologisch abbaubare Dura mater handelt, die durch das Verfahren gemäß einem der Ansprüche 3 bis 6 erhalten wurde, wobei sie optisch transparent ist und/oder eine Dicke von weniger als 80 µm, vorzugsweise zwischen 60 und 20 µm, aufweist.

9. Medizinisches Implantat, das das biologisch abbaubare Gewebe gemäß den Ansprüchen 7 oder 8 umfasst oder durch das Verfahren gemäß einem der Ansprüche 1 bis 6 erhalten wird.

10. Das medizinische Implantat gemäß Anspruch 9, wobei das medizinische Implantat aus einem beschichteten Stent oder einem Stentgraft ausgewählt ist.

11. Das medizinische Implantat nach Anspruch 9, wobei das medizinische Implantat ein kardiovaskuläres Implantat, eine endovaskuläre Prothese, eine Endoprothese, ein Ösophagusimplantat, ein Gallengangimplantat, ein dentales Implantat, ein orthopädisches Implantat, ein neurologisches Implantat, ein Implantat mit Mikrochip, ein beschichteter Stent, ein Stentgraft, ein Knochenimplantat, ein Glukosesensorimplantat, ein Neurostimulator, ein Cochlea-Implantat, eine Endoprothese zum Verschließen eines persistierenden Foramen ovale, eine Endoprothese zum Verschließen eines Vorhofseptumdefekts, eine Vorrichtung zum Verschließen des linken Vorhofohrs, ein Herzschrittmacher, ein leitungsloser Herzschrittmacher, ein Defibrillator, eine Herzklappenprothese, eine Venenklappe, ein Zahnimplantat.

12. Biologisch abbaubares biologisches Gewebe, erhalten durch ein Verfahren gemäß einem der Ansprüche 1 bis 6, zur Verwendung als medizinisches Implantat oder zur Verwendung in einem medizinischen Implantat.

13. Biologisch abbaubares biologisches Gewebe, erhalten durch ein Verfahren gemäß einem der Ansprüche 1 bis 6, zur Verwendung als Gewebepatch.

14. Biologisch abbaubare Dura mater, erhalten durch ein Verfahren gemäß einem der Ansprüche 3 bis 6, zur Verwendung als Gewebe-Patch oder zur Verwendung als röhrenförmiges medizinisches Implantat oder zur Reparatur von Defekten der Dura mater.

15. Vorrichtung zur Herstellung von biologisch abbaubarem Gewebe nach einem Verfahren gemäß einem der Ansprüche 1 bis 6, umfassend:
- mindestens eine Einheit zum Ausüben von Druck,
- eine oder mehrere Druckausgleichsschichten, die in der Einheit zum Ausüben von Druck angeordnet sind, und
- eine oder mehrere Schichten aus durchlässigem Material, die in der Einheit zum Ausüben von Druck angeordnet sind.

## Revendications

1. Procédé pour la préparation d'un tissu biodégradable, dans lequel le procédé comprend ou consiste en les étapes suivantes :
a) la fourniture d'un ou de plusieurs tissu(s) biologique(s) natif(s) ou d'un ou plusieurs tissu(s) biologique(s) décellularisé(s),
b) la stabilisation structurelle du ou des tissu(s) biologique(s) natif(s) ou du ou des tissu(s) biologique(s) décellularisé(s) avec un matériau de remplacement d'eau tissulaire pour obtenir un tissu structurellement stabilisé,
c) la fourniture d'au moins une, de préférence deux, couche(s) de matériau perméable,
d) le placement du tissu structurellement stabilisé sur, dans ou entre la ou les couche(s) de matériau perméable ;
e) la fourniture d'une ou plusieurs couches de compensation de pression et le recouvrement du tissu structurellement stabilisé ou de l'au moins une, de préférence deux, couche(s) de matériau perméable, avec les une ou plusieurs couches de compensation de pression ;
f) le séchage du tissu structurellement stabilisé tout en appliquant une pression sur le tissu structurellement stabilisé pour obtenir un tissu séché,
g) le retrait facultatif du tissu séché de la ou des couche(s) de matériau perméable et de la ou des couche(s) de compensation de pression facultative(s) ;

2. Procédé selon la revendication 1, dans lequel le tissu biologique natif est un tissu biologique contenant du collagène ou le tissu biologique décellularisé est basé sur un tissu biologique contenant du collagène.

3. Procédé selon la revendication 1, dans lequel le tissu biologique natif est de la dure-mère ou le tissu biologique décellularisé est basé sur de la dure-mère.

4. Procédé selon l'une quelconque des revendications précédentes, dans lequel l'au moins une, de préférence deux, couche(s) de matériau perméable comprennent ou consistent en un polymère.

5. Procédé selon l'une quelconque des revendications précédentes, dans lequel l'au moins une, de préférence deux, couche(s) de matériau perméable présente/présentent une taille de pore dans la plage de 10 à 60 µm.

6. Procédé selon l'une quelconque des revendications précédentes, dans lequel la couche de compensation de pression présente une dureté à la compression dans la plage de 20 kPa à 80 kPa, de préférence 30 kPa à 70 kPa, plus préférentiellement 40 kPa à 60 kPa, encore plus préférentiellement 60 kPa.

7. Tissu biodégradable obtenu par le procédé selon l'une des revendications précédentes.

8. Tissu biodégradable selon la revendication 7 qui est une dure-mère biodégradable obtenue par le procédé selon l'une des revendications 3 à 6, optiquement transparent et/ou ayant une épaisseur inférieure à 80 µm, de préférence comprise entre 60 et 20 µm.

9. Implant médical comprenant le tissu biodégradable selon les revendications 7 ou 8 ou obtenu par le procédé selon l'une quelconque des revendications 1 à 6.

10. Implant médical selon la revendication 9, dans lequel l'implant médical est choisi parmi un stent couvert ou une endoprothèse couverte.

11. Implant médical selon la revendication 9, dans lequel l'implant médical est un implant cardiovasculaire, une prothèse endovasculaire, une endoprothèse, un implant œsophagien, un implant biliaire, un implant dentaire, un implant orthopédique, un implant neurologique, un implant contenant une micropuce, un stent couvert, une endoprothèse couverte, un implant osseux, un implant capteur de glucose, un neurostimulateur, un implant cochléaire, une endoprothèse pour la fermeture d'un foramen ovale perméable, une endoprothèse pour la fermeture d'un défaut septal auriculaire, un dispositif de fermeture de l'appendice auriculaire gauche, un stimulateur cardiaque, un stimulateur cardiaque sans fil, un défibrillateur, une valve cardiaque prothétique, une valve veineuse, un implant de dent.

12. Tissu biologique biodégradable obtenu par un procédé selon l'une des revendications 1 à 6 pour une utilisation comme implant médical ou pour une utilisation dans un implant médical.

13. Tissu biologique biodégradable obtenu par un procédé selon l'une des revendications 1 à 6 pour une utilisation comme patch tissulaire.

14. Dure-mère biodégradable obtenue par un procédé selon l'une des revendications 3 à 6 pour une utilisation comme patch tissulaire ou pour une utilisation comme implant médical tubulaire ou pour une utilisation pour la réparation de défauts de la dure-mère.

15. Dispositif pour la préparation de tissu biodégradable selon un procédé selon l'une quelconque des revendications 1 à 6 comprenant :
- au moins une unité d'application de pression,
- une ou plusieurs couche(s) de compensation de pression agencée(s) dans l'unité d'application de pression et
- une ou plusieurs couche(s) de matériau perméable agencée(s) dans l'unité d'application de pression.
